# EUROPEAN PATENT APPLICATION

(11) **EP 4 722 199 A1**
(43) Date of publication of application: **08.04.2026**
(21) Application number: 24815402.3
(22) Date of filing: 24.05.2024
(51) Int. Cl.: C07D 249/14, C07D 257/06, C09D 5/00, C09D 7/63, C23C 26/00

(54) **AZOLE COMPOUND, METHOD FOR SYNTHESIZING SAID AZOLE COMPOUND, AND USE THEREOF**

(30) Priority: 30.05.2023 JP 2023088281
(71) Applicant: SHIKOKU CHEMICALS CORPORATION, Marugame-shi, Kagawa 763-8504 (JP)
(72) Inventor: TAKASAKU, Koji, Ayauta-gun, Kagawa 769-0202 (JP); MURAI, Takayuki, Ayauta-gun, Kagawa 769-0202 (JP); TANIOKA, Miya, Ayauta-gun, Kagawa 769-0202 (JP); KATSUMURA, Masato, Ayauta-gun, Kagawa 769-0202 (JP); OKADA, Kazuki, Ayauta-gun, Kagawa 769-0202 (JP); WATANABE, Natsuki, Ayauta-gun, Kagawa 769-0202 (JP)
(74) Representative: Ipsilon
(86) International application number: PCT/JP2024/019190
(87) International publication number: WO 2024/247919

(57) **Abstract**

The present invention provides a novel azole compound, a method for synthesizing the same, and a coupling agent. The azole compound according to the present invention is represented by chemical formula (I). The A in chemical formula (I) represents a group represented by any of formulas (1) through (3), and the substitution groups in formulas (1) through (3) are as defined in the description.

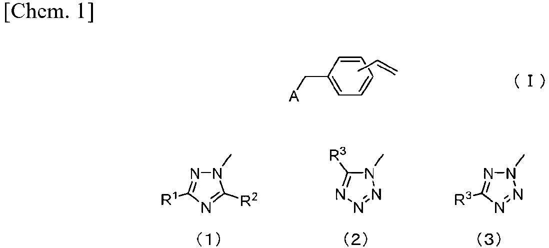

## Description

### TECHNICAL FIELD

The present invention relates to a novel azole compound, a method for synthesizing the azole compound, and use thereof.

### BACKGROUND ART

In the related art, coupling agents have been used to bond materials having different properties, such as organic materials and inorganic materials, and have been used in the fields of electronic materials, paints, primers, adhesives, and the like. Therefore, the coupling agents are essential agents for development and production of composite materials.

For example, a coupling agent (silane coupling agent) using a compound composed of an organic substance and silicon is known, and the silane coupling agent exhibits a function as an intermediate agent between an organic material and an inorganic material that are generally incompatible.

In addition, an azole compound such as a triazole compound has a function of preventing corrosion of a metal and a function of curing an epoxy resin or a urethane resin, and therefore, various coupling agents using an azole compound have been proposed.

As a coupling agent, for example, Patent Literature 1 proposes a triazole silane compound in which an alkoxysilyl group is introduced into a specific triazole ring. Patent Literature 2 proposes a triazole silane compound having specific two triazole rings and a disulfide bond (-S-S-), in which -CO-NH-(CH₂)m-Si(OR)₃ is introduced into the triazole ring.

### CITATION LIST

### PATENT LITERATURE

Patent Literature 1: WO 2018/186476
Patent Literature 2: WO 2015/002158

### SUMMARY OF INVENTION

### TECHNICAL PROBLEM

In recent years, in the field of electric and electronic materials, in order to meet the demands of miniaturization, thinning, precision, high-speed propagation, and the like, it is required to improve adhesion (adhesiveness) between materials. The azole compounds as disclosed in Patent Literature 1 and 2 can also exhibit excellent adhesion, but a compound capable of exhibiting higher adhesion has been required.

The present invention has been made in view of the above problems, and an object thereof is to provide a novel azole compound, a method for synthesizing the novel azole compound, and a coupling agent.

### SOLUTION TO PROBLEM

As a result of intensive studies to solve the above-described problems, the present inventors have found that the above-described problems can be solved by an azole compound obtained by reacting a certain kind of styryl compound and a certain kind of triazole compound or a certain kind of tetrazole compound, and have completed the present invention.

That is, a first aspect of the present invention provides an azole compound represented by the chemical formula (I).

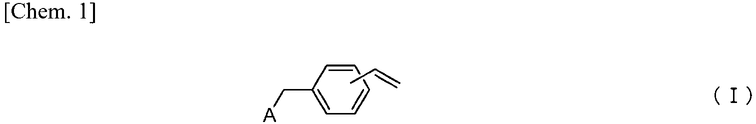

(In the formula (I), A represents a group represented by any of the formulas (1) to (3).)

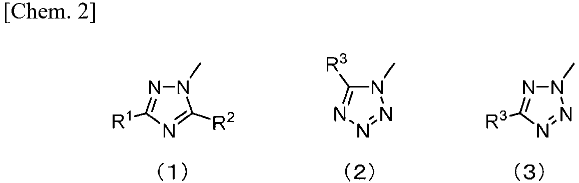

(In the formula (1), R¹ and R² are the same as or different from each other, and represent a hydrogen atom, a linear or branched alkyl group having 1 to 12 carbon atoms, an aryl group, an aralkyl group, an amino group which may have a substituent, a hydroxy group, a cyano group, a halogen atom, an alkylthio group having 1 to 6 carbon atoms, a group represented by the formula (11), or a group represented by the formula (12). In the formula (2) and the formula (3), R³ represents a hydrogen atom, a linear or branched alkyl group having 1 to 12 carbon atoms, an aryl group, an aralkyl group, an amino group which may have a substituent, a hydroxy group, a cyano group, a halogen atom, an alkylthio group having 1 to 6 carbon atoms, a group represented by the formula (21), or a group represented by the formula (31). Here, the case where R¹ and R² are hydrogen atoms at the same time, the case where R¹ and R² are groups represented by the formula (11) at the same time, the case where R¹ and R² are groups represented by the formula (12) at the same time, and the case where R¹ and R² are groups represented by the formula (11) and the formula (12) are excluded.)

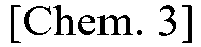

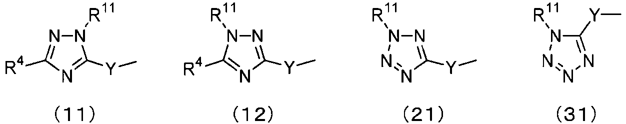

(In the formula (11) and the formula (12), R⁴ represents a hydrogen atom, a linear or branched alkyl group having 1 to 12 carbon atoms, an aryl group, an aralkyl group, an amino group which may have a substituent, a hydroxy group, a cyano group, a halogen atom, or an alkylthio group having 1 to 6 carbon atoms. In the formula (11), the formula (12), the formula (21), and the formula (31), R¹¹ represents a hydrogen atom or a group represented by the formula (4), Y represents a phenylene group, -NH-, or a group represented by -(CH₂)ₙ-, where n represents an integer of 0 to 12.)

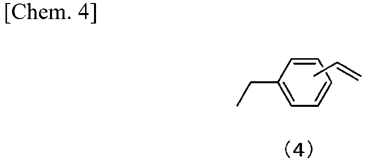

A second aspect of the present invention provides a method for synthesizing the azole compound according to the first aspect of the present invention, and the method includes reacting a styryl compound represented by the chemical formula (II) and a triazole compound represented by the chemical formula (III) or a tetrazole compound represented by the chemical formula (IV).

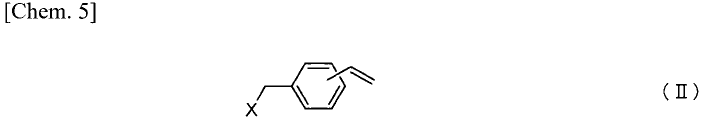

(In the formula (II), X represents a fluorine atom, a chlorine atom, a bromine atom, or an iodine atom.)

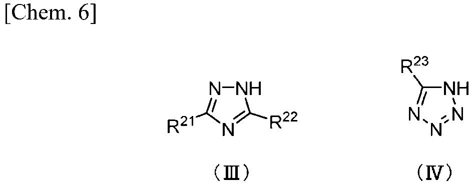

(In the formula (III), R²¹ and R²² are the same as or different from each other, and represent a hydrogen atom, a linear or branched alkyl group having 1 to 12 carbon atoms, an aryl group, an aralkyl group, an amino group which may have a substituent, a hydroxy group, a cyano group, a halogen atom, an alkylthio group having 1 to 6 carbon atoms, a group represented by the formula (13), or a group represented by the formula (14). In the formula (IV), R²³ represents a hydrogen atom, a linear or branched alkyl group having 1 to 12 carbon atoms, an aryl group, an aralkyl group, an amino group which may have a substituent, a hydroxy group, a cyano group, a halogen atom, an alkylthio group having 1 to 6 carbon atoms, a group represented by the formula (22), or a group represented by the formula (32). Here, the case where R²¹ and R²² are hydrogen atoms at the same time, the case where R²¹ and R²² are groups represented by the formula (13) at the same time, the case where R²¹ and R²² are groups represented by the formula (14) at the same time, and the case where R²¹ and R²² are groups represented by the formula (13) and the formula (14) are excluded.)

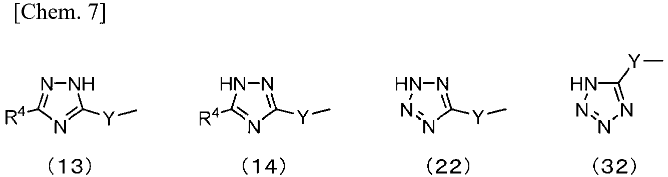

(R⁴ in the formula (13) and the formula (14), and Y in the formula (13), the formula (14), the formula (22), and the formula (32) are the same as those described above.)

A third aspect of the present invention provides a coupling agent containing the azole compound according to the first aspect of the present invention as a component.

A fourth aspect of the present invention provides a surface treatment liquid containing the azole compound according to the first aspect of the present invention.

A fifth aspect of the present invention is directed to the surface treatment liquid according to the fourth aspect of the present invention, in which the surface treatment liquid is used for treating a surface of at least one selected from the group consisting of a metal, an inorganic material, and a resin material.

A sixth aspect of the present invention is directed to the surface treatment liquid according to the fourth aspect of the present invention, in which the surface treatment liquid is used for adhering two materials selected from the group consisting of a metal, an inorganic material, and a resin material to each other.

A seventh aspect of the present invention is directed to the surface treatment liquid according to the fifth or sixth aspect of the present invention, in which the metal is at least one selected from the group consisting of copper, aluminum, titanium, nickel, tin, iron, silver, gold, and alloys thereof.

An eighth aspect of the present invention provides a surface treatment method including bringing the surface treatment liquid according to any one of the fourth to seventh aspects of the present invention into contact with a surface of at least one selected from the group consisting of a metal, an inorganic material, and a resin material.

A ninth aspect of the present invention is directed to the surface treatment method according to the eighth aspect of the present invention, in which the metal is at least one selected from the group consisting of copper, aluminum, titanium, nickel, tin, iron, silver, gold, and alloys thereof.

A tenth aspect of the present invention is directed to the surface treatment method according to the eighth aspect or the ninth aspect of the present invention, in which the metal is copper or a copper alloy.

An eleventh aspect of the present invention is directed to the surface treatment method according to any one of the eighth to tenth aspects of the present invention, in which an aqueous solution containing copper ions is brought into contact with a surface of copper or a copper alloy before the surface treatment liquid is brought into contact with the surface of the copper or the copper alloy.

A twelfth aspect of the present invention is directed to the surface treatment method according to any one of the eighth to eleventh aspects of the present invention, in which after the surface treatment liquid is brought into contact with the surface of the copper or the copper alloy, an acidic aqueous solution or an alkaline aqueous solution is brought into contact with the surface of the copper or the copper alloy.

A thirteenth aspect of the present invention provides an adhesion method including: bringing the surface treatment liquid according to any one of the fourth to seventh aspects of the present invention into contact with at least one selected from the group consisting of a metal, an inorganic material, and a resin material to form a chemical conversion film on the at least one; and adhering the at least one and another one of the group to each other via the chemical conversion film.

A fourteenth aspect of the present invention provides a method for adhering a metal and a resin material to each other, including: bringing the surface treatment liquid according to any one of the fourth to seventh aspects of the present invention into contact with at least one of the metal and the resin material to form a chemical conversion film on the at least one; and adhering the metal and the resin material to each other via the chemical conversion film.

A fifteenth aspect of the present invention provides a printed wiring board containing two materials selected from the group consisting of a metal, an inorganic material, and a resin material in which the two materials adhere to each other via a chemical conversion film formed by the surface treatment liquid according to any one of the fourth to seventh aspects of the present invention.

A sixteenth aspect of the present invention provides a semiconductor wafer containing two materials selected from the group consisting of a metal, an inorganic material, and a resin material in which the two materials adhere to each other via a chemical conversion film formed by the surface treatment liquid according to any one of the fourth to seventh aspects of the present invention.

A seventeenth aspect of the present invention provides an insulating composition containing the coupling agent according to the third aspect of the present invention and a resin material or an inorganic material.

An eighteenth aspect of the present invention provides an insulating material containing the insulating composition according to the seventeenth aspect of the present invention.

A nineteenth aspect of the present invention provides a printed wiring board having an insulating layer obtained from the insulating composition according to the seventeenth aspect of the present invention.

A twentieth aspect of the present invention provides a semiconductor wafer having an insulating layer obtained from the insulating composition according to the seventeenth aspect of the present invention.

### ADVANTAGEOUS EFFECTS OF INVENTION

The azole compound according to the present invention is a compound having an azole ring (a triazole ring or a tetrazole ring) together with a polymerizable group (-C₆H₄-CH=CH₂) in the molecule, and therefore, the azole compound has both a function of preventing a metal from rusting, which is a feature of the azole compound, and a function of curing an epoxy resin, a urethane resin, or the like. Therefore, the azole compound according to the present invention is useful as a component of a coupling agent or a surface treatment liquid.

In addition, the coupling agent according to the present invention contains, as a component, an azole compound having a polymerizable group (-C₆H₄-CH=CH₂), and therefore, the adhesion (adhesiveness) between materials having different properties can be enhanced. According to the surface treatment liquid containing the azole compound, it is possible to enhance the adhesion between two materials having different material types, that is, a metal and an inorganic material, a metal and a resin material, and an inorganic material and a resin material.

### DESCRIPTION OF EMBODIMENTS

Hereinafter, the present invention will be described in detail. The present invention is not limited to the embodiments described below.

### (Azole Compound)

An azole compound according to the present invention is represented by the chemical formula (I) (hereinafter, may be referred to as an azole compound according to the present invention).

The azole compound represented by the chemical formula (I) includes azole compounds respectively represented by the chemical formula (I-1) to the chemical formula (I-3).

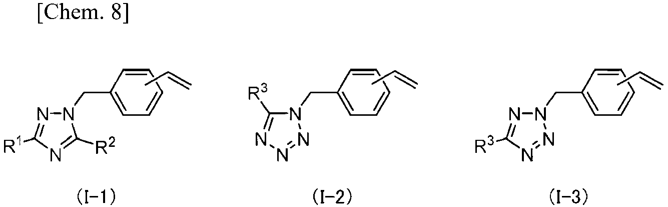

(In the formula (I-1), R¹ and R² are the same as or different from each other, and represent a hydrogen atom, a linear or branched alkyl group having 1 to 12 carbon atoms, an aryl group, an aralkyl group, an amino group which may have a substituent, a hydroxy group, a cyano group, a halogen atom, an alkylthio group having 1 to 6 carbon atoms, a group represented by the formula (11), or a group represented by the formula (12). In the formula (I-2) and the formula (I-3), R³ represents a hydrogen atom, a linear or branched alkyl group having 1 to 12 carbon atoms, an aryl group, an aralkyl group, an amino group which may have a substituent, a hydroxy group, a cyano group, a halogen atom, an alkylthio group having 1 to 6 carbon atoms, a group represented by the formula (21), or a group represented by the formula (31). Here, the case where R¹ and R² are hydrogen atoms at the same time, the case where R¹ and R² are groups represented by the formula (11) at the same time, the case where R¹ and R² are groups represented by the formula (12) at the same time, and the case where R¹ and R² are groups represented by the formula (11) and the formula (12) are excluded.)

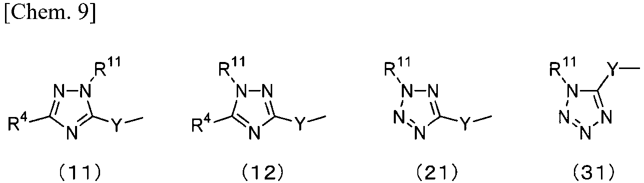

(In the formula (11) and the formula (12), R⁴ represents a hydrogen atom, a linear or branched alkyl group having 1 to 12 carbon atoms, an aryl group, an aralkyl group, an amino group which may have a substituent, a hydroxy group, a cyano group, a halogen atom, or an alkylthio group having 1 to 6 carbon atoms. In the formula (11), the formula (12), the formula (21), and the formula (31), R¹¹ represents a hydrogen atom or a group represented by the formula (4), Y represents a phenylene group, -NH-, or a group represented by -(CH₂)ₙ-, where n represents an integer of 0 to 12.)

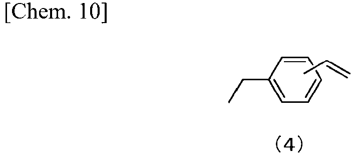

Specific examples of the linear alkyl group having 1 to 12 carbon atoms, which is represented by R¹, R², and R³, include a methyl group, an ethyl group, an n-propyl group, an n-butyl group, an n-pentyl group, an n-hexyl group, a heptyl group, an octyl group, a nonyl group, a decyl group, an undecyl group, and a dodecyl group.

The branched alkyl group having 1 to 12 carbon atoms is a branched alkyl group having 3 to 12 carbon atoms, and specific examples thereof include an isopropyl group, an isobutyl group, an s-butyl group, a t-butyl group, an isopentyl group, an s-pentyl group, a t-pentyl group, an isohexyl group, an s-hexyl group, and a t-hexyl group.

Specific examples of the aryl group include a phenyl group, an o-tolyl group, an m-tolyl group, a p-tolyl group, a xylyl group, a trimethylphenyl group, a tetramethylphenyl group, a 1-naphthyl group, and a 2-naphthyl group.

Specific examples of the aralkyl group include a benzyl group, a phenylethyl group, a phenylpropyl group, and a methylbenzyl group.

Specific examples of the amino group which may have a substituent include an amino group, a methylamino group, an ethylamino group, a propylamino group, an isopropylamino group, a phenylamino group, an acetamide group, a guanidino group, a dimethylamino group, a diethylamino group, a dipropylamino group, a diisopropylamino group, an ethylmethylamino group, and a diphenylamino group.

Specific examples of the halogen atom include a fluorine atom, a chlorine atom, a bromine atom, and an iodine atom.

Specific examples of the alkylthio group having 1 to 6 carbon atoms include a methylthio group, an ethylthio group, a propylthio group, an isopropylthio group, a butylthio group, an isobutylthio group, a sec-butylthio group, a tert-butylthio group, a pentylthio group, and a hexylthio group.

Specific examples of the linear alkyl group having 1 to 12 carbon atoms, the branched alkyl group having 1 to 12 carbon atoms, the aryl group, the aralkyl group, the amino group which may have a substituent, the halogen atom, and the alkylthio group having 1 to 6 carbon atoms represented by R⁴ in the group represented by the formula (11) and the group represented by the formula (12) are also the same as those described above.

In the formula (I-1), R¹ and R² are the same as or different from each other and are more preferably an amino group or an alkylthio group having 1 to 6 carbon atoms, and still more preferably a primary amino group or an alkylthio group having 1 to 4 carbon atoms.

In the formula (I-2) or the formula (I-3), R³ is more preferably an amino group or an alkylthio group having 1 to 6 carbon atoms, and still more preferably a primary amino group or an alkylthio group having 1 to 4 carbon atoms.

Specific examples of the azole compound represented by the chemical formula (I-1) include:
3-methyl-1-(4-vinylbenzyl)-1H-1,2,4-triazole,
5-methyl-1-(4-vinylbenzyl)-1H-1,2,4-triazole,
3-ethyl-1-(4-vinylbenzyl)-1H-1,2,4-triazole,
5-ethyl-1-(4-vinylbenzyl)-1H-1,2,4-triazole,
3-propyl-1-(4-vinylbenzyl)-1H-1,2,4-triazole,
5-propyl-1-(4-vinylbenzyl)-1H-1,2,4-triazole,
3-isopropyl-1-(4-vinylbenzyl)-1H-1,2,4-triazole,
5-isopropyl-1-(4-vinylbenzyl)-1H-1,2,4-triazole,
3-butyl-1-(4-vinylbenzyl)-1H-1,2,4-triazole,
5-butyl-1-(4-vinylbenzyl)-1H-1,2,4-triazole,
3-hexyl-1-(4-vinylbenzyl)-1H-1,2,4-triazole,
5-hexyl-1-(4-vinylbenzyl)-1H-1,2,4-triazole,
3-octyl-1-(4-vinylbenzyl)-1H-1,2,4-triazole,
5-octyl-1-(4-vinylbenzyl)-1H-1,2,4-triazole,
3-decyl-1-(4-vinylbenzyl)-1H-1,2,4-triazole,
5-decyl-1-(4-vinylbenzyl)-1H-1,2,4-triazole,
3-dodecyl-1-(4-vinylbenzyl)-1H-1,2,4-triazole,
5-dodecyl-1-(4-vinylbenzyl)-1H-1,2,4-triazole,
3,5-dimethyl-1-(4-vinylbenzyl)-1H-1,2,4-triazole,
3,5-diisopropyl-1-(4-vinylbenzyl)-1H-1,2,4-triazole,
3-methyl-5-octyl-1-(4-vinylbenzyl)-1H-1,2,4-triazole,
5-methyl-3-octyl-1-(4-vinylbenzyl)-1H-1,2,4-triazole,
3-ethyl-5-octyl-1-(4-vinylbenzyl)-1H-1,2,4-triazole,
5-ethyl-3-octyl-1-(4-vinylbenzyl)-1H-1,2,4-triazole,
3-phenyl-1-(4-vinylbenzyl)-1H-1,2,4-triazole,
5-phenyl-1-(4-vinylbenzyl)-1H-1,2,4-triazole,
3-methyl-5-phenyl-1-(4-vinylbenzyl)-1H-1,2,4-triazole,
5-methyl-3-phenyl-1-(4-vinylbenzyl)-1H-1,2,4-triazole,
3-ethyl-5-phenyl-1-(4-vinylbenzyl)-1H-1,2,4-triazole,
5-ethyl-3-phenyl-1-(4-vinylbenzyl)-1H-1,2,4-triazole,
3,5-diphenyl-1-(4-vinylbenzyl)-1H-1,2,4-triazole,
3-benzyl-1-(4-vinylbenzyl)-1H-1,2,4-triazole,
5-benzyl-1-(4-vinylbenzyl)-1H-1,2,4-triazole,
5-propyl-3-benzyl-1-(4-vinylbenzyl)-1H-1,2,4-triazole,
3-propyl-5-benzyl-1-(4-vinylbenzyl)-1H-1,2,4-triazole,
3-benzyl-5-phenyl-1-(4-vinylbenzyl)-1H-1,2,4-triazole,
5-benzyl-3-phenyl-1-(4-vinylbenzyl)-1H-1,2,4-triazole,
3-amino-1-(4-vinylbenzyl)-1H-1,2,4-triazole,
5-amino-1-(4-vinylbenzyl)-1H-1,2,4-triazole,
3-amino-5-ethyl-1-(4-vinylbenzyl)-1H-1,2,4-triazole,
5-amino-3-ethyl-1-(4-vinylbenzyl)-1H-1,2,4-triazole,
3-amino-5-phenyl-1-(4-vinylbenzyl)-1H-1,2,4-triazole,
5-amino-3-phenyl-1-(4-vinylbenzyl)-1H-1,2,4-triazole,
3-amino-5-benzyl-1-(4-vinylbenzyl)-1H-1,2,4-triazole,
5-amino-3-benzyl-1-(4-vinylbenzyl)-1H-1,2,4-triazole,
3,5-di-amino-1-(2-vinylbenzyl)-1H-1,2,4-triazole,
3,5-di-amino-1-(3-vinylbenzyl)-1H-1,2,4-triazole,
3,5-di-amino-1-(4-vinylbenzyl)-1H-1,2,4-triazole,
3-methylthio-1-(4-vinylbenzyl)-1H-1,2,4-triazole,
5-methylthio-1-(4-vinylbenzyl)-1H-1,2,4-triazole,
3-ethylthio-5-isopropyl-1-(4-vinylbenzyl)-1H-1,2,4-triazole,
5-ethylthio-3-isopropyl-1-(4-vinylbenzyl)-1H-1,2,4-triazole,
3-isopropylthio-1-(4-vinylbenzyl)-1H-1,2,4-triazole,
5-isopropylthio-1-(4-vinylbenzyl)-1H-1,2,4-triazole,
3-hexylthio-1-(4-vinylbenzyl)-1H-1,2,4-triazole,
5-hexylthio-1-(4-vinylbenzyl)-1H-1,2,4-triazole,
3,5-bis(methylthio)-1-(4-vinylbenzyl)-1H-1,2,4-triazole,
3-methylthio-5-hexylthio-1-(4-vinylbenzyl)-1H-1,2,4-triazole,
5-methylthio-3-hexylthio-1-(4-vinylbenzyl)-1H-1,2,4-triazole,
3-amino-5-methylthio-1-(4-vinylbenzyl)-1H-1,2,4-triazole,
5-amino-3-methylthio-1-(4-vinylbenzyl)-1H-1,2,4-triazole,
3-amino-5-isopropylthio-1-(4-vinylbenzyl)-1H-1,2,4-triazole,
5-amino-3-isopropylthio-1-(4-vinylbenzyl)-1H-1,2,4-triazole,
3-amino-5-hexylthio-1-(4-vinylbenzyl)-1H-1,2,4-triazole,
5-amino-3-hexylthio-1-(4-vinylbenzyl)-1H-1,2,4-triazole,
3-methylamino-1-(4-vinylbenzyl)-1H-1,2,4-triazole,
5-methylamino-1-(4-vinylbenzyl)-1H-1,2,4-triazole,
3,5-bis(methylamino)-1-(4-vinylbenzyl)-1H-1,2,4-triazole,
3-amino-5-methylamino-1-(4-vinylbenzyl)-1H-1,2,4-triazole,
5-amino-3-methylamino-1-(4-vinylbenzyl)-1H-1,2,4-triazole,
3-methylamino-5-ethyl-1-(4-vinylbenzyl)-1H-1,2,4-triazole,
5-methylamino-3-ethyl-1-(4-vinylbenzyl)-1H-1,2,4-triazole,
3-methylamino-5-phenyl-1-(4-vinylbenzyl)-1H-1,2,4-triazole,
5-methylamino-3-phenyl-1-(4-vinylbenzyl)-1H-1,2,4-triazole,
3-methylamino-5-benzyl-1-(4-vinylbenzyl)-1H-1,2,4-triazole,
5-methylamino-3-benzyl-1-(4-vinylbenzyl)-1H-1,2,4-triazole,
3-phenylamino-1-(4-vinylbenzyl)-1H-1,2,4-triazole,
5-phenylamino-1-(4-vinylbenzyl)-1H-1,2,4-triazole,
3,5-bis(phenylamino)-1-(4-vinylbenzyl)-1H-1,2,4-triazole,
3-amino-5-phenylamino-1-(4-vinylbenzyl)-1H-1,2,4-triazole,
5-amino-3-phenylamino-1-(4-vinylbenzyl)-1H-1,2,4-triazole,
3-phenylamino-5-ethyl-1-(4-vinylbenzyl)-1H-1,2,4-triazole,
5-phenylamino-3-ethyl-1-(4-vinylbenzyl)-1H-1,2,4-triazole,
3-phenylamino-5-phenyl-1-(4-vinylbenzyl)-1H-1,2,4-triazole,
5-phenylamino-3-phenyl-1-(4-vinylbenzyl)-1H-1,2,4-triazole,
3-phenylamino-5-benzyl-1-(4-vinylbenzyl)-1H-1,2,4-triazole,
5-phenylamino-3-benzyl-1-(4-vinylbenzyl)-1H-1,2,4-triazole,
3-acetamide-1-(4-vinylbenzyl)-1H-1,2,4-triazole,
5-acetamide-1-(4-vinylbenzyl)-1H-1,2,4-triazole,
3,5-bis(acetamide)-1-(4-vinylbenzyl)-1H-1,2,4-triazole,
3-amino-5-acetamide-1-(4-vinylbenzyl)-1H-1,2,4-triazole,
5-amino-3-acetamide-1-(4-vinylbenzyl)-1H-1,2,4-triazole,
3-acetamide-5-ethyl-1-(4-vinylbenzyl)-1H-1,2,4-triazole,
5-acetamide-3-ethyl-1-(4-vinylbenzyl)-1H-1,2,4-triazole,
3-acetamide-5-phenyl-1-(4-vinylbenzyl)-1H-1,2,4-triazole,
5-acetamide-3-phenyl-1-(4-vinylbenzyl)-1H-1,2,4-triazole,
3-acetamide-5-benzyl-1-(4-vinylbenzyl)-1H-1,2,4-triazole,
5-acetamide-3-benzyl-1-(4-vinylbenzyl)-1H-1,2,4-triazole,
3-guanidino-1-(4-vinylbenzyl)-1H-1,2,4-triazole,
5-guanidino-1-(4-vinylbenzyl)-1H-1,2,4-triazole,
3,5-bis(guanidino)-1-(4-vinylbenzyl)-1H-1,2,4-triazole,
3-amino-5-guanidino-1-(4-vinylbenzyl)-1H-1,2,4-triazole,
5-amino-3-guanidino-1-(4-vinylbenzyl)-1H-1,2,4-triazole,
3-guanidino-5-ethyl-1-(4-vinylbenzyl)-1H-1,2,4-triazole,
5-guanidino-3-ethyl-1-(4-vinylbenzyl)-1H-1,2,4-triazole,
3-guanidino-5-phenyl-1-(4-vinylbenzyl)-1H-1,2,4-triazole,
5-guanidino-3-phenyl-1-(4-vinylbenzyl)-1H-1,2,4-triazole,
3-guanidino-5-benzyl-1-(4-vinylbenzyl)-1H-1,2,4-triazole,
5-guanidino-3-benzyl-1-(4-vinylbenzyl)-1H-1,2,4-triazole,
3-hydroxy-1-(4-vinylbenzyl)-1H-1,2,4-triazole,
5-hydroxy-1-(4-vinylbenzyl)-1H-1,2,4-triazole,
3-hydroxy-5-ethyl-1-(4-vinylbenzyl)-1H-1,2,4-triazole,
5-hydroxy-3-ethyl-1-(4-vinylbenzyl)-1H-1,2,4-triazole,
3-hydroxy-5-phenyl-1-(4-vinylbenzyl)-1H-1,2,4-triazole,
5-hydroxy-3-phenyl-1-(4-vinylbenzyl)-1H-1,2,4-triazole,
3-hydroxy-5-benzyl-1-(4-vinylbenzyl)-1H-1,2,4-triazole,
5-hydroxy-3-benzyl-1-(4-vinylbenzyl)-1H-1,2,4-triazole,
3-cyano-1-(4-vinylbenzyl)-1H-1,2,4-triazole,
5-cyano-1-(4-vinylbenzyl)-1H-1,2,4-triazole,
3-cyano-5-ethyl-1-(4-vinylbenzyl)-1H-1,2,4-triazole,
5-cyano-3-ethyl-1-(4-vinylbenzyl)-1H-1,2,4-triazole,
3-cyano-5-phenyl-1-(4-vinylbenzyl)-1H-1,2,4-triazole,
5-cyano-3-phenyl-1-(4-vinylbenzyl)-1H-1,2,4-triazole,
3-cyano-5-benzyl-1-(4-vinylbenzyl)-1H-1,2,4-triazole,
5-cyano-3-benzyl-1-(4-vinylbenzyl)-1H-1,2,4-triazole,
3-chloro-1-(4-vinylbenzyl)-1H-1,2,4-triazole,
5-chloro-1-(4-vinylbenzyl)-1H-1,2,4-triazole,
3-chloro-5-ethyl-1-(4-vinylbenzyl)-1H-1,2,4-triazole,
5-chloro-3-ethyl-1-(4-vinylbenzyl)-1H-1,2,4-triazole,
3-bromo-5-phenyl-1-(4-vinylbenzyl)-1H-1,2,4-triazole,
5-bromo-3-phenyl-1-(4-vinylbenzyl)-1H-1,2,4-triazole,
3-iodo-5-benzyl-1-(4-vinylbenzyl)-1H-1,2,4-triazole,
5-iodo-3-benzyl-1-(4-vinylbenzyl)-1H-1,2,4-triazole,
1-(4-vinylbenzyl)-3,3'-(1,4-phenylene)bis(1H-1,2,4-triazole),
1-(4-vinylbenzyl)-3,3'-(1,2-phenylene)bis(5-methyl-1,2,4-1H-triazole),
1-(4-vinylbenzyl)-3,3'-(1,3-phenylene)bis(5-butyl-1,2,4-1H-triazole),
1-(4-vinylbenzyl)-3,3'-(1,4-phenylene)bis(5-amino-1,2,4-1H-triazole),
1-(4-vinylbenzyl)-3,3'-(1,4-phenylene)bis(5-ethylthio-1H-1,2,4-triazole),
1-(4-vinylbenzyl)-3,3'-iminobis(5-ethyl-1H-1,2,4-triazole),
1-(4-vinylbenzyl)-3,3'-iminobis(5-p-tolyl-1H-1,2,4-triazole),
1-(4-vinylbenzyl)-3,3'-iminobis(5-amino-1H-1,2,4-triazole),
1-(4-vinylbenzyl)-3,3'-iminobis(5-isopropyl-1H-1,2,4-triazole),
1-(4-vinylbenzyl)-3,3'-bis(5-methyl-1H-1,2,4-triazole),
1-(4-vinylbenzyl)-3,3'-bis(5-pentyl-1H-1,2,4-triazole),
1-(4-vinylbenzyl)-3,3'-bis(5-amino-1H-1,2,4-triazole),
1-(4-vinylbenzyl)-3,3'-bis(5-phenyl-1H-1,2,4-triazole),
1-(4-vinylbenzyl)-5-amino-5'-methyl-3,3'-bi-1H-1,2,4-triazole,
1-(4-vinylbenzyl)-3,3'-methylenebis(5-isobutyl-1H-1,2,4-triazole),
1-(4-vinylbenzyl)-3,3'-methylenebis(5-hydroxy-1H-1,2,4-triazole),
1-(4-vinylbenzyl)-3,3'-methylenebis(5-amino-1H-1,2,4-triazole),
1-(4-vinylbenzyl)-3,3'-ethylenebis(5-ethyl-1H-1,2,4-triazole),
1-(4-vinylbenzyl)-3,3'-ethylenebis(5-amino-1H-1,2,4-triazole),
1-(4-vinylbenzyl)-3,3'-tetramethylenebis(5-benzyl-1,2,4-1H-triazole),
1-(4-vinylbenzyl)-3,3'-tetramethylenebis(5-butylthio-1H-1,2,4-triazole),
1-(4-vinylbenzyl)-3,3'-tetramethylenebis(5-amino-1H-1,2,4-triazole),
1-(4-vinylbenzyl)-3,3'-pentamethylenebis(1H-1,2,4-triazole),
1-(4-vinylbenzyl)-3,3'-pentamethylenebis(5-amino-1H-1,2,4-triazole),
1-(4-vinylbenzyl)-3,3'-hexamethylenebis(5-amino-1H-1,2,4-triazole),
1-(4-vinylbenzyl)-3,3'-octamethylenebis(5-amino-1H-1,2,4-triazole),
1-(4-vinylbenzyl)-3,3'-nonamethylenebis(5-amino-1H-1,2,4-triazole),
1-(4-vinylbenzyl)-3,3'-decamethylenebis(5-amino-1H-1,2,4-triazole),
1-(4-vinylbenzyl)-3,3'-undecamethylenebis(5-amino-1H-1,2,4-triazole),
1-(4-vinylbenzyl)-3,3'-dodecamethylenebis(5-amino-1H-1,2,4-triazole),
3,3'-(1,4-phenylene)bis[1-(4-vinylbenzyl)-1H-1,2,4-triazole],
3,3'-(1,2-phenylene)bis[1-(4-vinylbenzyl)-5-methyl-1H-1,2,4-triazole],
3,3'-(1,3-phenylene)bis{1-(4-vinylbenzyl)-5-butyl-1H-1,2,4-triazole],
3,3'-(1,4-phenylene)bis[1-(4-vinylbenzyl)-5-amino-1H-1,2,4-triazole],
3,3'-(1,4-phenylene)bis[1-(4-vinylbenzyl)-5-ethylthio-1H-1,2,4-triazole],
3,3'-iminobis[1-(4-vinylbenzyl)-5-ethyl-1H-1,2,4-triazole],
3,3'-iminobis[1-(4-vinylbenzyl)-5-p-tolyl-1H-1,2,4-triazole],
3,3'-iminobis[1-(4-vinylbenzyl)-5-amino-1H-1,2,4-triazole],
3,3'-iminobis[1-(4-vinylbenzyl)-5-isopropyl-1H-1,2,4-triazole],
3,3'-bis[1-(4-vinylbenzyl)-1H-1,2,4-triazole],
3,3'-bis[1-(4-vinylbenzyl)-5-pentyl-1H-1,2,4-triazole],
3,3'-bis[1-(4-vinylbenzyl)-5-amino-1H-1,2,4-triazole],
3,3'-bis[1-(4-vinylbenzyl)-5-phenyl-1H-1,2,4-triazole],
1,1'-bis(4-vinylbenzyl)-5-amino-5'-methyl-3,3'-bi-1H-1,2,4-triazole,
3,3'-methylenebis[1-(4-vinylbenzyl)-5-isobutyl-1H-1,2,4-triazole],
3,3'-methylenebis[1-(4-vinylbenzyl)-5-hydroxy-1H-1,2,4-triazole],
3,3'-methylenebis[1-(4-vinylbenzyl)-5-amino-1H-1,2,4-triazole],
3,3'-ethylenebis[1-(4-vinylbenzyl)-5-ethyl-1H-1,2,4-triazole],
3,3'-ethylenebis[1-(4-vinylbenzyl)-5-amino-1H-1,2,4-triazole],
3,3'-tetramethylenebis[1-(4-vinylbenzyl)-5-benzyl-1,2,4-1H-triazole],
3,3'-tetramethylenebis[1-(4-vinylbenzyl)-5-butylthio-1H-1,2,4-triazole],
3,3'-tetramethylenebis[1-(4-vinylbenzyl)-5-amino-1H-1,2,4-triazole],
3,3'-pentamethylenebis[1-(4-vinylbenzyl)-1H-1,2,4-triazole],
3,3'-pentamethylenebis[1-(4-vinylbenzyl)-5-amino-1H-1,2,4-triazole],
3,3'-hexamethylenebis[1-(4-vinylbenzyl)-5-amino-1H-1,2,4-triazole],
3,3'-octamethylenebis[1-(4-vinylbenzyl)-5-amino-1H-1,2,4-triazole],
3,3'-nonamethylenebis[1-(4-vinylbenzyl)-5-amino-1H-1,2,4-triazole],
3,3'-decamethylenebis[1-(4-vinylbenzyl)-5-amino-1H-1,2,4-triazole],
3,3'-undecamethylenebis[1-(4-vinylbenzyl)-5-amino-1H-1,2,4-triazole], and
3,3'-dodecamethylenebis[1-(4-vinylbenzyl)-5-amino-1H-1,2,4-triazole].

Specific examples of the azole compound represented by the chemical formula (I-2) include:
1-(4-vinylbenzyl)-1H-tetrazole,
5-methyl-1-(4-vinylbenzyl)-1H-tetrazole,
5-ethyl-1-(4-vinylbenzyl)-1H-tetrazole,
5-propyl-1-(4-vinylbenzyl)-1H-tetrazole,
5-isopropyl-1-(4-vinylbenzyl)-1H-tetrazole,
5-butyl-1-(4-vinylbenzyl)-1H-tetrazole,
5-tert-butyl-1-(4-vinylbenzyl)-1H-tetrazole,
5-pentyl-1-(4-vinylbenzyl)-1H-tetrazole,
5-hexyl-1-(4-vinylbenzyl)-1H-tetrazole,
5-octyl-1-(4-vinylbenzyl)-1H-tetrazole,
5-decyl-1-(4-vinylbenzyl)-1H-tetrazole,
5-dodecyl-1-(4-vinylbenzyl)-1H-tetrazole,
5-phenyl-1-(4-vinylbenzyl)-1H-tetrazole,
5-(p-tolyl)-1-(4-vinylbenzyl)-1H-tetrazole,
5-benzyl-1-(4-vinylbenzyl)-1H-tetrazole,
5-phenethyl-1-(4-vinylbenzyl)-1H-tetrazole,
5-methylthio-1-(4-vinylbenzyl)-1H-tetrazole,
5-ethylthio-1-(4-vinylbenzyl)-1H-tetrazole,
5-propylthio-1-(4-vinylbenzyl)-1H-tetrazole,
5-isopropylthio-1-(4-vinylbenzyl)-1H-tetrazole,
5-butylthio-1-(4-vinylbenzyl)-1H-tetrazole,
5-pentylthio-1-(4-vinylbenzyl)-1H-tetrazole,
5-hexylthio-1-(4-vinylbenzyl)-1H-tetrazole,
5-amino-1-(2-vinylbenzyl)-1H-tetrazole,
5-amino-1-(3-vinylbenzyl)-1H-tetrazole,
5-amino-1-(4-vinylbenzyl)-1H-tetrazole,
5-methylamino-1-(4-vinylbenzyl)-1H-tetrazole,
5-ethylamino-1-(4-vinylbenzyl)-1H-tetrazole,
5-phenylamino-1-(4-vinylbenzyl)-1H-tetrazole,
5-acetamide-1-(4-vinylbenzyl)-1H-tetrazole,
5-guanidino-1-(4-vinylbenzyl)-1H-tetrazole,
5-hydroxy-1-(4-vinylbenzyl)-1H-tetrazole,
5-cyano-1-(4-vinylbenzyl)-1H-tetrazole,
5-bromo-1-(4-vinylbenzyl)-1H-tetrazole,
1-(4-vinylbenzyl)-5,5'-(1,4-phenylene)bis(1H-tetrazole),
1-(4-vinylbenzyl)-5,5'-(1,2-phenylene)bis(1H-tetrazole),
1-(4-vinylbenzyl)-5,5'-(1,3-phenylene)bis(1H-tetrazole),
1-(4-vinylbenzyl)-5,5'-iminobis(1H-tetrazole),
1-(4-vinylbenzyl)-5,5'-bi-1H-tetrazole,
1-(4-vinylbenzyl)-5,5'-methylenebis(1H-tetrazole),
1-(4-vinylbenzyl)-5,5'-ethylenebis(1H-tetrazole),
1-(4-vinylbenzyl)-5,5'-tetramethylenebis(1H-tetrazole),
1-(4-vinylbenzyl)-5,5'-pentamethylenebis(1H-tetrazole),
1-(4-vinylbenzyl)-5,5'-hexamethylenebis(1H-tetrazole),
1-(4-vinylbenzyl)-5,5'-octamethylenebis(1H-tetrazole),
1-(4-vinylbenzyl)-5,5'-nonamethylenebis(1H-tetrazole),
1-(4-vinylbenzyl)-5,5'-decamethylenebis(1H-tetrazole),
1-(4-vinylbenzyl)-5,5'-undecamethylenebis(1H-tetrazole),
1-(4-vinylbenzyl)-5,5'-dodecamethylenebis(1H-tetrazole),
5,5'-(1,4-phenylene)bis[1-(4-vinylbenzyl)-1H-tetrazole],
5,5'-(1,2-phenylene)bis[1-(4-vinylbenzyl)-1H-tetrazole],
5,5'-(1,3-phenylene)bis[1-(4-vinylbenzyl)-1H-tetrazole],
5,5'-iminobis[1-(4-vinylbenzyl)-1H-tetrazole],
5,5'-bis[1-(4-vinylbenzyl)-1H-tetrazole],
1-(4-vinylbenzyl)-2'-(4-vinylbenzyl)-bi-1,2,3,4-tetrazole,
5,5'-methylenebis[1-(4-vinylbenzyl)-1H-tetrazole],
5,5'-ethylenebis[1-(4-vinylbenzyl)-1H-tetrazole],
5,5'-tetramethylenebis[1-(4-vinylbenzyl)-1H-tetrazole],
5,5'-pentamethylenebis[1-(4-vinylbenzyl)-1H-tetrazole],
5,5'-hexamethylenebis[1-(4-vinylbenzyl)-1H-tetrazole],
5,5'-octamethylenebis[1-(4-vinylbenzyl)-1H-tetrazole],
5,5'-nonamethylenebis[1-(4-vinylbenzyl)-1H-tetrazole],
5,5'-decamethylenebis[1-(4-vinylbenzyl)-1H-tetrazole],
5,5'-undecamethylenebis[1-(4-vinylbenzyl)-1H-tetrazole], and
5,5'-dodecamethylenebis[1-(4-vinylbenzyl)-1H-tetrazole].

Specific examples of the azole compound represented by the chemical formula (I-3) include:
2-(4-vinylbenzyl)-2H-tetrazole,
5-methyl-2-(4-vinylbenzyl)-2H-tetrazole,
5-ethyl-2-(4-vinylbenzyl)-2H-tetrazole,
5-propyl-2-(4-vinylbenzyl)-2H-tetrazole,
5-isopropyl-2-(4-vinylbenzyl)-2H-tetrazole,
5-butyl-2-(4-vinylbenzyl)-2H-tetrazole,
5-tert-butyl-2-(4-vinylbenzyl)-2H-tetrazole,
5-pentyl-2-(4-vinylbenzyl)-2H-tetrazole,
5-hexyl-2-(4-vinylbenzyl)-2H-tetrazole,
5-octyl-2-(4-vinylbenzyl)-2H-tetrazole,
5-decyl-2-(4-vinylbenzyl)-2H-tetrazole,
5-dodecyl-2-(4-vinylbenzyl)-2H-tetrazole,
5-phenyl-2-(4-vinylbenzyl)-2H-tetrazole,
5-(p-tolyl)-2-(4-vinylbenzyl)-2H-tetrazole,
5-benzyl-2-(4-vinylbenzyl)-2H-tetrazole,
5-phenethyl-2-(4-vinylbenzyl)-2H-tetrazole,
5-methylthio-2-(4-vinylbenzyl)-2H-tetrazole,
5-ethylthio-2-(4-vinylbenzyl)-2H-tetrazole,
5-propylthio-2-(4-vinylbenzyl)-2H-tetrazole,
5-isopropylthio-2-(4-vinylbenzyl)-2H-tetrazole,
5-butylthio-2-(4-vinylbenzyl)-2H-tetrazole,
5-pentylthio-2-(4-vinylbenzyl)-2H-tetrazole,
5-hexylthio-2-(4-vinylbenzyl)-2H-tetrazole,
5-amino-2-(2-vinylbenzyl)-2H-tetrazole,
5-amino-2-(3-vinylbenzyl)-2H-tetrazole,
5-amino-2-(4-vinylbenzyl)-2H-tetrazole,
5-methylamino-2-(4-vinylbenzyl)-2H-tetrazole,
5-ethylamino-2-(4-vinylbenzyl)-2H-tetrazole,
5-phenylamino-2-(4-vinylbenzyl)-2H-tetrazole,
5-acetamide-2-(4-vinylbenzyl)-2H-tetrazole,
5-guanidino-2-(4-vinylbenzyl)-2H-tetrazole,
5-hydroxy-2-(4-vinylbenzyl)-2H-tetrazole,
5-cyano-2-(4-vinylbenzyl)-2H-tetrazole,
5-bromo-2-(4-vinylbenzyl)-2H-tetrazole,
2-(4-vinylbenzyl)-5,5'-(1,4-phenylene)bis(2H-tetrazole),
2-(4-vinylbenzyl)-5,5'-(1,2-phenylene)bis(2H-tetrazole),
2-(4-vinylbenzyl)-5,5'-(1,3-phenylene)bis(2H-tetrazole),
2-(4-vinylbenzyl)-5,5'-iminobis(2H-tetrazole),
2-(4-vinylbenzyl)-5,5'-bi-2H-tetrazole,
2-(4-vinylbenzyl)-5,5'-methylenebis(2H-tetrazole),
2-(4-vinylbenzyl)-5,5'-ethylenebis(2H-tetrazole),
2-(4-vinylbenzyl)-5,5'-tetramethylenebis(2H-tetrazole),
2-(4-vinylbenzyl)-5,5'-pentamethylenebis(2H-tetrazole),
2-(4-vinylbenzyl)-5,5'-hexamethylenebis(2H-tetrazole),
2-(4-vinylbenzyl)-5,5'-octamethylenebis(2H-tetrazole),
2-(4-vinylbenzyl)-5,5'-nonamethylenebis(2H-tetrazole),
2-(4-vinylbenzyl)-5,5'-decamethylenebis(2H-tetrazole),
2-(4-vinylbenzyl)-5,5'-undecamethylenebis(2H-tetrazole),
2-(4-vinylbenzyl)-5,5'-dodecamethylenebis(2H-tetrazole),
5,5'-(1,4-phenylene)bis[2-(4-vinylbenzyl)-2H-tetrazole],
5,5'-(1,2-phenylene)bis[2-(4-vinylbenzyl)-2H-tetrazole],
5,5'-(1,3-phenylene)bis[2-(4-vinylbenzyl)-2H-tetrazole],
5,5'-iminobis[2-(4-vinylbenzyl)-2H-tetrazole],
5,5'-bis[2-(4-vinylbenzyl)-2H-tetrazole],
5,5'-methylenebis[2-(4-vinylbenzyl)-2H-tetrazole],
5,5'-ethylenebis[2-(4-vinylbenzyl)-2H-tetrazole],
5,5'-tetramethylenebis[2-(4-vinylbenzyl)-2H-tetrazole],
5,5'-pentamethylenebis[2-(4-vinylbenzyl)-2H-tetrazole],
5,5'-hexamethylenebis[2-(4-vinylbenzyl)-2H-tetrazole],
5,5'-octamethylenebis[2-(4-vinylbenzyl)-2H-tetrazole],
5,5'-nonamethylenebis[2-(4-vinylbenzyl)-2H-tetrazole],
5,5'-decamethylenebis[2-(4-vinylbenzyl)-2H-tetrazole],
5,5'-undecamethylenebis[2-(4-vinylbenzyl)-2H-tetrazole], and
5,5'-dodecamethylenebis[2-(4-vinylbenzyl)-2H-tetrazole].

The azole compound according to the present invention can be obtained by reacting a styryl compound represented by the chemical formula (II) and a triazole compound represented by the chemical formula (III) or a tetrazole compound represented by the chemical formula (IV).

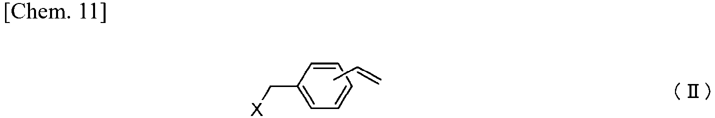

(In the formula (II), X represents a fluorine atom, a chlorine atom, a bromine atom, or an iodine atom.)

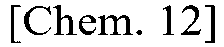

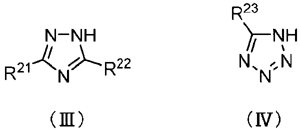

(In the formula (III), R²¹ and R²² are the same as or different from each other, and represent a hydrogen atom, a linear or branched alkyl group having 1 to 12 carbon atoms, an aryl group, an aralkyl group, an amino group which may have a substituent, a hydroxy group, a cyano group, a halogen atom, an alkylthio group having 1 to 6 carbon atoms, a group represented by the formula (13), or a group represented by the formula (14). In the formula (IV), R²³ represents a hydrogen atom, a linear or branched alkyl group having 1 to 12 carbon atoms, an aryl group, an aralkyl group, an amino group which may have a substituent, a hydroxy group, a cyano group, a halogen atom, an alkylthio group having 1 to 6 carbon atoms, a group represented by the formula (22), or a group represented by the formula (32). Here, the case where R²¹ and R²² are hydrogen atoms at the same time, the case where R²¹ and R²² are groups represented by the formula (13) at the same time, the case where R²¹ and R²² are groups represented by the formula (14) at the same time, and the case where R²¹ and R²² are groups represented by the formula (13) and the formula (14) are excluded.)

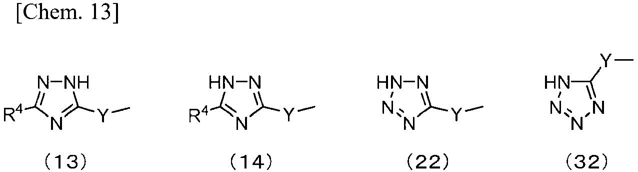

(In the formula (13) and the formula (14), R⁴ represents a hydrogen atom, a linear or branched alkyl group having 1 to 12 carbon atoms, an aryl group, an aralkyl group, an amino group which may have a substituent, a hydroxy group, a cyano group, a halogen atom, or an alkylthio group having 1 to 6 carbon atoms, and in the formula (13), the formula (14), the formula (22), and the formula (32), Y represents a phenylene group, -NH-, or a group represented by -(CH₂)ₙ-, where n represents an integer of 0 to 12.)

X in Formula (II) is a fluorine atom, a chlorine atom, a bromine atom, or an iodine atom, and is preferably a chlorine atom or a bromine atom from the viewpoint of reactivity and availability of raw materials.

In the chemical formulas (III) and (IV), specific examples of the linear alkyl group having 1 to 12 carbon atoms, the branched alkyl group having 1 to 12 carbon atoms, the aryl group, the aralkyl group, the amino group which may have a substituent, the halogen atom, and the alkylthio group having 1 to 6 carbon atoms, which are represented by R²¹, R²², and R²³, are the same as those shown in the chemical formula (I), and preferable examples thereof are also the same.

Examples of the styryl compound represented by the chemical formula (II) include:
2-vinylbenzyl chloride,
3-vinylbenzyl chloride,
4-vinylbenzyl chloride,
2-vinylbenzyl bromide,
3-vinylbenzyl bromide,
4-vinylbenzyl bromide,
2-vinylbenzyl iodide,
3-vinylbenzyl iodide, and
4-vinylbenzyl iodide.

These exemplified styryl compounds can be used by purchasing commercially available reagents, or can be synthesized in accordance with, for example, a synthesis method described in WO 1998/028264.

Examples of the triazole compound represented by the chemical formula (III) include:
3-methyl-1H-1,2,4-triazole,
3-ethyl-1H-1,2,4-triazole,
3-propyl-1H-1,2,4-triazole,
3-isopropyl-1H-1,2,4-triazole,
3-butyl-1H-1,2,4-triazole,
3-hexyl-1H-1,2,4-triazole,
3-octyl-1H-1,2,4-triazole,
3-decyl-1H-1,2,4-triazole,
3-dodecyl-1H-1,2,4-triazole,
3,5-dimethyl-1H-1,2,4-triazole,
3,5-diisopropyl-1H-1,2,4-triazole,
3-ethyl-5-octyl-1H-1,2,4-triazole,
5-methyl-3-octyl-1H-1,2,4-triazole,
3-phenyl-1H-1,2,4-triazole,
3-methyl-5-phenyl-1H-1,2,4-triazole,
3-ethyl-5-phenyl-1H-1,2,4-triazole,
3,5-diphenyl-1H-1,2,4-triazole,
3-benzyl-1H-1,2,4-triazole,
5-propyl-3-benzyl-1H-1,2,4-triazole,
3-benzyl-5-phenyl-1H-1,2,4-triazole,
3-amino-1H-1,2,4-triazole,
3-amino-5-ethyl-1H-1,2,4-triazole,
3-amino-5-phenyl-1H-1,2,4-triazole,
3-amino-5-benzyl-1H-1,2,4-triazole,
3,5-diamino-1H-1,2,4-triazole,
3-methylthio-1H-1,2,4-triazole,
3-ethylthio-5-isopropyl-1H-1,2,4-triazole,
3-isopropylthio-1H-1,2,4-triazole,
3-hexylthio-1H-1,2,4-triazole,
3,5-bis(methylthio)-1H-1,2,4-triazole,
3-methylthio-5-hexylthio-1H-1,2,4-triazole,
3-amino-5-methylthio-1H-1,2,4-triazole,
3-amino-5-isopropylthio-1H-1,2,4-triazole,
3-amino-5-hexylthio-1H-1,2,4-triazole,
3-methylamino-1H-1,2,4-triazole,
3,5-bis(methylamino)-1H-1,2,4-triazole,
3-amino-5-methylamino-1H-1,2,4-triazole,
3-methylamino-5-ethyl-1H-1,2,4-triazole,
3-methylamino-5-phenyl-1H-1,2,4-triazole,
3-methylamino-5-benzyl-1H-1,2,4-triazole,
3-phenylamino-1H-1,2,4-triazole,
3,5-bis(phenylamino)-1H-1,2,4-triazole,
3-amino-5-phenylamino-1H-1,2,4-triazole,
3-phenylamino-5-ethyl-1H-1,2,4-triazole,
3-phenylamino-5-phenyl-1H-1,2,4-triazole,
3-phenylamino-5-benzyl-1H-1,2,4-triazole,
3-acetamide-1H-1,2,4-triazole,
3,5-bis(acetamide)-1H-1,2,4-triazole,
3-amino-5-acetamide-1H-1,2,4-triazole,
3-acetamide-5-ethyl-1H-1,2,4-triazole,
3-acetamide-5-phenyl-1H-1,2,4-triazole,
3-acetamide-5-benzyl-1H-1,2,4-triazole,
3-guanidino-1H-1,2,4-triazole,
3,5-bis(guanidino)-1H-1,2,4-triazole,
3-amino-5-guanidino-1H-1,2,4-triazole,
3-guanidino-5-ethyl-1H-1,2,4-triazole,
3-guanidino-5-phenyl-1H-1,2,4-triazole,
3-guanidino-5-benzyl-1H-1,2,4-triazole,
3-hydroxy-1H-1,2,4-triazole,
3-hydroxy-5-ethyl-1H-1,2,4-triazole,
3-hydroxy-5-phenyl-1H-1,2,4-triazole,
3-hydroxy-5-benzyl-1H-1,2,4-triazole,
3-cyano-1H-1,2,4-triazole,
3-cyano-5-ethyl-1H-1,2,4-triazole,
3-cyano-5-phenyl-1H-1,2,4-triazole,
3-cyano-5-benzyl-1H-1,2,4-triazole,
3-chloro-1H-1,2,4-triazole,
3-chloro-5-ethyl-1H-1,2,4-triazole,
3-bromo-5-phenyl-1H-1,2,4-triazole,
3-iodo-5-benzyl-1H-1,2,4-triazole,
3,3'-(1,4-phenylene)bis(1H-1,2,4-triazole),
3,3'-(1,4-phenylene)bis(5-methyl-1H-1,2,4-triazole),
3,3'-(1,2-phenylene)bis(5-ethyl-1H-1,2,4-triazole),
3,3'-(1,4-phenylene)bis(5-isopropyl-1H-1,2,4-triazole),
3,3'-(1,3-phenylene)bis(5-butyl-1H-1,2,4-triazole),
3,3'-(1,4-phenylene)bis(5-phenyl-1H-1,2,4-triazole),
3,3'-(1,4-phenylene)bis(5-amino-1H-1,2,4-triazole),
3,3'-(1,4-phenylene)bis(5-hydroxy-1H-1,2,4-triazole),
3,3'-(1,4-phenylene)bis(5-ethylthio-1H-1,2,4-triazole),
5-amino-3,3'-(1,4-phenylene)bis(1H-1,2,4-triazole),
3,3'-iminobis(1H-1,2,4-triazole),
3,3'-iminobis(5-methyl-1H-1,2,4-triazole),
3,3'-iminobis(5-hexyl-1H-1,2,4-triazole),
3,3'-iminobis(5-phenyl-1H-1,2,4-triazole),
3,3'-iminobis(5-benzyl-1H-1,2,4-triazole),
3,3'-iminobis(5-amino-1H-1,2,4-triazole),
3,3'-iminobis(5-hydroxy-1H-1,2,4-triazole),
3,3'-iminobis(5-methylthio-1H-1,2,4-triazole),
5-amino-3,3'-iminobis(1H-1,2,4-triazole),
3,3'-bi-1H-1,2,4-triazole,
3,3'-bis(5-methyl-1H-1,2,4-triazole),
3,3'-bis(5-ethyl-1H-1,2,4-triazole),
3,3'-bis(5-pentyl-1H-1,2,4-triazole),
3,3'-bis(5-p-tolyl-1H-1,2,4-triazole),
3,3'-bis(5-benzyl-1H-1,2,4-triazole),
3,3'-bis(5-amino-1H-1,2,4-triazole),
3,3'-bis(5-hydroxy-1H-1,2,4-triazole),
3,3'-bis(5-hexylthio-1H-1,2,4-triazole),
5-amino-5'-methyl-3,3'-bi-1H-1,2,4-triazole,
3,3'-methylenebis(1H-1,2,4-triazole),
3,3'-methylenebis(5-methyl-1H-1,2,4-triazole),
3,3'-methylenebis(5-isobutyl-1H-1,2,4-triazole),
3,3'-methylenebis(5-phenyl-1H-1,2,4-triazole),
3,3'-methylenebis(5-phenethyl-1H-1,2,4-triazole),
3,3'-methylenebis(5-amino-1H-1,2,4-triazole),
3,3'-methylenebis(5-hydroxy-1H-1,2,4-triazole),
3,3'-methylenebis(5-methylthio-1H-1,2,4-triazole),
5-amino-5'-ethyl-3,3'-methylenebis(1H-1,2,4-triazole),
3,3'-ethylenebis(1H-1,2,4-triazole),
3,3'-ethylenebis(5-propyl-1H-1,2,4-triazole),
3,3'-ethylenebis(5-hexyl-1H-1,2,4-triazole),
3,3'-ethylenebis(5-m-tolyl-1H-1,2,4-triazole),
3,3'-ethylenebis(5-amino-1H-1,2,4-triazole),
3,3'-ethylenebis(5-hydroxy-1H-1,2,4-triazole),
3,3'-ethylenebis(5-isopropylthio-1H-1,2,4-triazole),
5-amino-3,3'-ethylenebis(1H-1,2,4-triazole),
3,3'-trimethylenebis(1H-1,2,4-triazole),
5-methyl-3,3'-trimethylenebis(1H-1,2,4-triazole),
3,3'-trimethylenebis(5-butyl-1H-1,2,4-triazole),
3,3'-trimethylenebis(5-phenyl-1H-1,2,4-triazole),
3,3'-trimethylenebis(5-amino-1H-1,2,4-triazole),
3,3'-trimethylenebis(5-propylthio-1H-1,2,4-triazole),
5-amino-5'-benzyl-3,3'-trimethylenebis(1H-1,2,4-triazole),
3,3'-tetramethylenebis(1H-1,2,4-triazole),
3,3'-tetramethylenebis(5-ethyl-1H-1,2,4-triazole),
3,3'-tetramethylenebis(5-hexyl-1H-1,2,4-triazole),
3,3'-tetramethylenebis(5-benzyl-1H-1,2,4-triazole),
3,3'-tetramethylenebis(5-amino-1H-1,2,4-triazole),
3,3'-tetramethylenebis(5-hydroxy-1H-1,2,4-triazole),
3,3'-tetramethylenebis(5-butylthio-1H-1,2,4-triazole),
5-amino-3,3'-tetramethylenebis(1H-1,2,4-triazole),
3,3'-pentamethylenebis(1H-1,2,4-triazole),
3,3'-pentamethylenebis(5-propyl-1H-1,2,4-triazole),
3,3'-pentamethylenebis(5-amino-1H-1,2,4-triazole),
3,3'-pentamethylenebis(5-hydroxy-1H-1,2,4-triazole),
3,3'-pentamethylenebis(5-pentylthio-1H-1,2,4-triazole),
5-amino-5'-phenyl-3,3'-pentamethylenebis(1H-1,2,4-triazole),
3,3'-hexamethylenebis(1H-1,2,4-triazole),
3,3'-hexamethylenebis(5-pentyl-1H-1,2,4-triazole),
3,3'-hexamethylenebis(5-amino-1H-1,2,4-triazole),
5-hydroxy-3,3'-hexamethylenebis(1H-1,2,4-triazole),
5-phenyl-3,3'-hexamethylenebis(1H-1,2,4-triazole),
5-amino-5'-methylthio-3,3'-hexamethylenebis(1H-1,2,4-triazole),
3,3'-heptamethylenebis(1H-1,2,4-triazole),
3,3'-heptamethylenebis(5-tert-butyl-1H-1,2,4-triazole),
3,3'-heptamethylenebis(5-phenyl-1H-1,2,4-triazole),
3,3'-heptamethylenebis(5-amino-1H-1,2,4-triazole),
5-amino-5'-hydroxy-3,3'-heptamethylenebis(1H-1,2,4-triazole),
3,3'-octamethylenebis(1H-1,2,4-triazole),
3,3'-octamethylenebis(5-isobutyl-1H-1,2,4-triazole),
3,3'-octamethylenebis(5-amino-1H-1,2,4-triazole),
3,3'-octamethylenebis(5-hydroxy-1H-1,2,4-triazole),
5-propylthio-3,3'-octamethylenebis(1H-1,2,4-triazole),
3,3'-nonamethylenebis(1H-1,2,4-triazole),
3,3'-nonamethylenebis(5-isopentyl-1H-1,2,4-triazole),
5-phenyl-3,3'-nonamethylenebis(1H-1,2,4-triazole),
3,3'-nonamethylenebis(5-amino-1H-1,2,4-triazole),
3,3'-nonamethylenebis(5-methylthio-1H-1,2,4-triazole),
3,3'-decamethylenebis(1H-1,2,4-triazole),
3,3'-decamethylenebis(5-methyl-1H-1,2,4-triazole),
3,3'-decamethylenebis(5-amino-1H-1,2,4-triazole),
5-ethyl-3,3'-decamethylenebis(1H-1,2,4-triazole),
3,3'-undecamethylenebis(1H-1,2,4-triazole),
3,3'-undecamethylenebis(5-propyl-1H-1,2,4-triazole),
3,3'-undecamethylenebis(5-amino-1H-1,2,4-triazole),
3,3'-undecamethylenebis(5-methylthio-1H-1,2,4-triazole),
3,3'-dodecamethylenebis(1H-1,2,4-triazole),
3,3'-dodecamethylenebis(5-ethyl-1H-1,2,4-triazole),
3,3'-dodecamethylenebis(5-amino-1H-1,2,4-triazole), and
3,3'-undecamethylenebis(5-hydroxy-1H-1,2,4-triazole).

These triazole compounds exemplified above can be used by purchasing commercially available reagents, or can be synthesized in accordance with, for example, synthesis methods described in J. Org. Chem., Vol. 44, p. 4160 (1979), US 5,098,920B, Chem. Eur., Vol. 18, p. 16742 (2012), J. Applied. Chem., Vol. 82, p. 276 (2009), and US 2,744,116B.

Examples of the tetrazole compound represented by the chemical formula (IV) include:
1H-tetrazole,
5-methyl-1H-tetrazole,
5-ethyl-1H-tetrazole,
5-propyl-1H-tetrazole,
5-isopropyl-1H-tetrazole,
5-butyl-1H-tetrazole,
5-tert-butyl-1H-tetrazole,
5-pentyl-1H-tetrazole,
5-hexyl-1H-tetrazole,
5-octyl-1H-tetrazole,
5-decyl-1H-tetrazole,
5-dodecyl-1H-tetrazole,
5-phenyl-1H-tetrazole,
5-(p-tolyl)-1H-tetrazole,
5-benzyl-1H-tetrazole,
5-phenethyl-1H-tetrazole,
5-methylthio-1H-tetrazole,
5-ethylthio-1H-tetrazole,
5-propylthio-1H-tetrazole,
5-isopropylthio-1H-tetrazole,
5-butylthio-1H-tetrazole,
5-pentylthio-1H-tetrazole,
5-hexylthio-1H-tetrazole,
5-amino-1H-tetrazole,
5-methylamino-1H-tetrazole,
5-ethylamino-1H-tetrazole,
5-phenylamino-1H-tetrazole,
5-acetamide-1H-tetrazole,
5-guanidino-1H-tetrazole,
5-hydroxy-1H-tetrazole,
5-cyano-1H-tetrazole,
5-bromo-1H-tetrazole,
5,5'-(1,4-phenylene)bis(1H-tetrazole),
5,5'-(1,2-phenylene)bis(1H-tetrazole),
5,5'-(1,3-phenylene)bis(1H-tetrazole),
5,5'-iminobis(1H-tetrazole),
5,5'-bi(1H-tetrazole),
5,5'-methylenebis(1H-tetrazole),
5,5'-ethylenebis(1H-tetrazole),
5,5'-trimethylenebis(1H-tetrazole),
5,5'-tetramethylenebis(1H-tetrazole),
5,5'-pentamethylenebis(1H-tetrazole),
5,5'-hexamethylenebis(1H-tetrazole),
5,5'-heptamethylenebis(1H-tetrazole),
5,5'-octamethylenebis(1H-tetrazole),
5,5'-nonamethylenebis(1H-tetrazole),
5,5'-decamethylenebis(1H-tetrazole),
5,5'-undecamethylenebis(1H-tetrazole), and
5,5'-dodecamethylenebis(1H-tetrazole).

These tetrazole compounds exemplified above can be used by purchasing commercially available reagents, or can be synthesized in accordance with, for example, synthesis methods described in CN 109503505 and WO 2015/084830.

The azole compound according to the present invention can be synthesized, for example, by reacting a styryl compound represented by the chemical formula (II) and a triazole compound represented by the chemical formula (III) or a tetrazole compound represented by the chemical formula (IV) in the presence of a dehydrohalogenation agent in an appropriate amount of a reaction solvent at an appropriate reaction temperature for an appropriate reaction time. The following reaction scheme (A) shows a reaction scheme for obtaining an azole compound (I-1) in which A is a group represented by the formula (1).

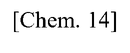

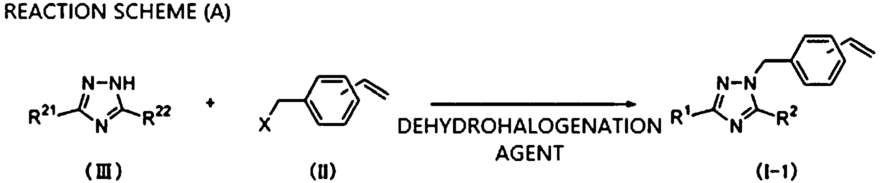

(R¹ and R² in the formula (I-1), X in the formula (II), and R²¹ and R²² in the formula (III) are the same as those described above.)

The reaction solvent is not particularly limited as long as it is a solvent inert to the styryl compound represented by the chemical formula (II) and the triazole compound represented by the chemical formula (III) or the tetrazole compound represented by the chemical formula (IV), and examples thereof include:
hydrocarbon solvents such as hexane, toluene, and xylene;
ether solvents such as diethyl ether, tetrahydrofuran, dioxane, and cyclopentyl methyl ether;
ester solvents such as ethyl acetate and butyl acetate;
alcohol solvents such as methanol and ethanol;
amide solvents such as N,N-dimethylformamide, N,N-dimethylacetamide, and N-methylpyrrolidone;
ketone solvents such as acetone, methyl ethyl ketone, methyl isobutyl ketone, and cyclohexanone;
acetonitrile, dimethyl sulfoxide, hexamethylphosphoramide, and the like.

Examples of the dehydrohalogenation agent include:
alkali metal alkoxides such as sodium methoxide, sodium ethoxide, potassium methoxide, and potassium t-butoxide;
alkali carbonate salts such as sodium carbonate and potassium carbonate;
organic bases such as triethylamine and diazabicycloundecene;
sodium hydride, and the like.

In this reaction, the usage amount (charged amount) of the styryl compound represented by the chemical formula (II) with respect to the usage amount (charged amount) of the triazole compound represented by the chemical formula (III) or the tetrazole compound represented by the chemical formula (IV) is preferably 0.8 to 2.4 equivalents in consideration of factors such as reaction temperature, reaction time, as well as the type of the raw material and the reaction solvent to be used, and the reaction scale.

In addition, the dehydrohalogenation agent is used to neutralize the hydrogen halide produced as a by-product in this reaction, and therefore, the usage amount (charged amount) thereof may be equal to or more than the usage amount of the styryl compound represented by the chemical formula (II).

The reaction temperature is not particularly limited as long as it falls within a temperature range in which -NH of the azole ring of the triazole compound represented by the chemical formula (III) or that of the tetrazole compound represented by the chemical formula (IV) reacts with the styryl compound represented by the chemical formula (II), and is preferably in a range of 0°C to 150°C and more preferably in a range of 5°C to 100°C.

The reaction time is appropriately determined according to the set reaction temperature, and is preferably in a range of 30 minutes to 48 hours, and more preferably in a range of 1 hour to 24 hours.

### (Coupling Agent)

The coupling agent according to the present invention contains the azole compound represented by the chemical formula (I) as a component.

In the case where the coupling agent according to the present invention is used, the same surface treatment method as in the case of a coupling agent according to the related art can be adopted.

Examples of the surface treatment method include (a) a method of spray-coating a base material with a treatment liquid obtained by diluting an appropriate amount of the coupling agent with an organic solvent, (b) a method of spray-coating a base material with a treatment liquid obtained by diluting the coupling agent with water and an organic solvent, (c) a method of spray-coating a base material with a treatment liquid obtained by diluting the coupling agent with water, (d) a method of immersing a base material in the treatment liquid obtained by diluting the coupling agent with an organic solvent, (e) a method of immersing a base material in the treatment liquid obtained by diluting the coupling agent with water and an organic solvent, and (f) a method of immersing a base material in the treatment liquid obtained by diluting the coupling agent with water.

Examples of the organic solvent include:
hydrocarbon solvents such as benzene, toluene, xylene, heptane, hexane, cyclohexane, and n-octane;
halogenated hydrocarbon solvents such as dichloromethane, dichloroethane, carbon tetrachloride, chloroform, chlorobenzene, dichlorobenzene, and trichlorobenzene;
ketone solvents such as acetone, methyl ethyl ketone, and methyl isobutyl ketone;
ether solvents such as diethyl ether, tetrahydrofuran, dioxane, ethylene glycol monomethyl ether (methyl cellosolve), ethylene glycol monoethyl ether (ethyl cellosolve), ethylene glycol monobutyl ether, and diethylene glycol monobutyl ether; and
alcohol solvents such as methanol, ethanol, 1-propanol, 2-propanol, n-butyl alcohol, 2-butyl alcohol, tert-butyl alcohol, ethylene glycol, diethylene glycol, and propylene glycol.

Examples of the base material used in the present invention include granular, needle-shaped, fibrous, woven, plate-shaped, foil-shaped, and amorphous base materials formed of a metal, an inorganic material, a resin material, or the like.

Examples of the metal include copper, aluminum, titanium, nickel, tin, iron, silver, gold, and alloys thereof, and a plate, a foil, or a plating film made of these metals can be used as a base material.

In the case of a copper alloy, a specific example of the alloy is not particularly limited as long as the alloy is an alloy containing copper, and examples thereof include alloys such as a Cu-Ag alloy, a Cu-Te alloy, a Cu-Mg alloy, a Cu-Sn alloy, a Cu-Si alloy, a Cu-Mn alloy, a Cu-Be-Co alloy, a Cu-Ti alloy, a Cu-Ni-Si alloy, a Cu-Zn-Ni alloy, a Cu-Cr alloy, a Cu-Zr alloy, a Cu-Fe alloy, a Cu-Al alloy, a Cu-Zn alloy, and a Cu-Co alloy.

Examples of other alloys include an aluminum alloy (Al-Si alloy), a nickel alloy (Ni-Cr alloy), and an iron alloy (Fe-Ni alloy, stainless steel, steel).

Among these metals, copper and a copper alloy are preferable.

Examples of the inorganic material include silicon, ceramic, an inorganic material used as a filler, and glass.

Specific examples thereof include: silicon compounds such as silicon, silicon carbide, silica, glass, diatomaceous earth, calcium silicate, talc, glass beads, sericite activated clay, bentonite, aluminosilicate, and mica; oxides such as alumina, zinc oxide, iron oxide, magnesium oxide, tin oxide, and titanium oxide; hydroxides such as magnesium hydroxide, aluminum hydroxide, and basic magnesium carbonate; carbonates such as calcium carbonate, zinc carbonate, hydrotalcite, and magnesium carbonate; sulfates such as barium sulfate and gypsum; titanates such as barium titanate; nitrides such as aluminum nitride and silicon nitride; and carbon fibers.

Among these inorganic materials, silicon, ceramics (alumina, silicon carbide, aluminum nitride, silicon nitride, barium titanate, and the like), and glass are preferable.

Examples of the resin material include nylon, an acrylate resin, an epoxy resin, a polybenzoxazole resin, a silicone resin, a polyimide resin, a bismaleimide resin, a maleimide resin, a cyanate resin, a polyphenylene ether resin, a polyphenylene oxide resin, a polybutadiene resin, an olefin resin, a fluorine-containing resin, a polyetherimide resin, a polyether ether ketone resin, and a liquid crystal resin, and these resins may be mixed or modified with each other to form a combination.

Among these resin materials, a polyphenylene ether resin, a polyphenylene oxide resin, a liquid crystal resin, an acrylate resin, an epoxy resin, an olefin resin, a polybenzoxazole resin, a silicone resin, and a polyimide resin are preferable.

By subjecting the base material to the surface treatment with the treatment liquid containing the coupling agent of the present invention, the lipophilicity of the surface of the base material can be increased, and the affinity (adhesion, adhesiveness) to a resin or the like can be improved.

In order to further exhibit the effect of this treatment, the surface-treated base material may be further heat-treated.

### (Insulating Composition)

An insulating composition can be formed when a resin material or an inorganic material contains the coupling agent of the present invention.

The insulating composition can also be obtained by dissolving the azole compound represented by the chemical formula (I) in an organic solvent or the like and mixing the resultant with a resin material or an inorganic material.

The content of the azole compound represented by the chemical formula (I) in the insulating composition is preferably 0.001 wt% to 10 wt%, and more preferably 0.01 wt% to 5 wt%. In the case where the content of the azole compound represented by the chemical formula (I) is 0.001 wt% or more in the insulating composition, the effect of improving the adhesion is sufficiently obtained, and in the case where this concentration exceeds 10 wt%, the effect of improving the adhesion substantially reaches a peak. Therefore, from an economical viewpoint, the content of the azole compound represented by the chemical formula (I) is preferably 10 wt% or less.

The insulating composition can be produced by a known method. For example, the insulating composition can be produced by dissolving the azole compound represented by the chemical formula (I) in an organic solvent and mixing the resulting solution with a solid or liquid resin material. In addition, the azole compound represented by the chemical formula (I) may be directly added to and mixed with a liquid resin material to produce the insulating composition.

The insulating composition according to the present invention forms an insulating material having high adhesive strength, and therefore, the insulating composition can be suitably used for various electrical and electronic components and electronic devices such as printed wiring boards.

JP 2009-19266A describes an invention related to a method for forming a silane coupling agent film. The method includes a step of applying a liquid containing a silane coupling agent to a metal surface, a step of drying the metal surface to which the liquid is applied at a temperature of 25°C to 150°C for 5 minutes or shorter, and a step of washing the dried metal surface with water.

In addition, it is said that an adhesive metal layer made of tin or the like may be formed on the metal surface in advance by an immersion plating solution as a surface treatment.

The surface treatment liquid according to the present invention can be used in place of the liquid containing the silane coupling agent. It should be noted that the matters described in this patent publication are incorporated herein by reference.

### (Surface Treatment Liquid)

The surface treatment liquid according to the present invention contains the azole compound represented by the chemical formula (I).

The surface treatment liquid according to the present invention can be prepared by mixing the azole compound with water and/or a solubilizing agent.

The water used for preparing the surface treatment liquid is preferably pure water such as ion exchange water or distilled water.

Examples of the solubilizing agent used for preparing the surface treatment liquid include an acid, an alkali, and an organic solvent. One kind of these solubilizing agents may be used alone, or two or more kinds thereof may be used in combination.

In the surface treatment liquid according to the present invention, it is preferable to use a solubilizing agent and water in order to promote dissolution (solution formation) of the azole compound.

Examples of the acid include inorganic acids such as hydrochloric acid, sulfuric acid, nitric acid, and phosphoric acid; and organic acids such as formic acid, acetic acid, propionic acid, butyric acid, 2-ethylbutyric acid, valeric acid, caproic acid, enanthic acid, caprylic acid, pelargonic acid, capric acid, lauric acid, myristic acid, palmitic acid, margaric acid, oleic acid, stearic acid, glycolic acid, lactic acid, gluconic acid, glyceric acid, malonic acid, succinic acid, levulinic acid, benzoic acid, oxalic acid, tartaric acid, malic acid, benzenesulfonic acid, tosylic acid, methanesulfonic acid, 5-sulfosalicylic acid, 4-hydroxybenzenesulfonic acid, 3-methyl-4-hydroxybenzenesulfonic acid, 4-aminobenzenesulfonic acid, camphorsulfonic acid, benzenedisulfonic acid, benzenetrisulfonic acid, sulfamic acid, and amino acids. One kind of these acids may be used alone, or two or more kinds thereof may be used in combination.

Examples of the alkali include hydroxides of alkali metals, such as sodium hydroxide and potassium hydroxide, and amines such as ammonia, methylamine, dimethylamine, trimethylamine, ethylamine, diethylamine, triethylamine, propylamine, isopropylamine, butylamine, pentylamine, hexylamine, heptylamine, octylamine, nonylamine, allylamine, ethylenediamine, diethylenetriamine, triethylenetetramine, monoethanolamine, diethanolamine, triethanolamine, monopropanolamine, dipropanolamine, tripropanolamine, monoisopropanolamine, diisopropanolamine, triisopropanolamine, 2-amino-1-propanol, N,N-dimethylethanolamine, cyclohexylamine, aniline, pyrrolidine, piperidine, piperazine, and pyridine. One kind of these alkalis may be used alone, or two or more kinds thereof may be used in combination.

Examples of the organic solvent include methanol, ethanol, 1-propanol, 2-propanol, butanol, tert-butyl alcohol, ethylene glycol, propylene glycol, 1,4-butanediol, glycerin, diethylene glycol, triethylene glycol, ethylene glycol monomethyl ether, ethylene glycol dimethyl ether, ethylene glycol monoethyl ether, ethylene glycol diethyl ether, ethylene glycol monobutyl ether, propylene glycol monomethyl ether, propylene glycol monoethyl ether, propylene glycol monobutyl ether, diethylene glycol monomethyl ether, diethylene glycol dimethyl ether, diethylene glycol monoethyl ether, diethylene glycol diethyl ether, diethylene glycol monobutyl ether, triethylene glycol dimethyl ether, triethylene glycol diethyl ether, tetrahydrofurfuryl alcohol, furfuryl alcohol, acetone, tetrahydrofuran, dioxane, acetonitrile, 2-pyrrolidone, formamide, dimethylformamide, dimethylacetamide, dimethyl sulfoxide, sulfolane, dimethyl carbonate, ethylene carbonate, N-methylpyrrolidone, γ-butyrolactone, and 1,3-dimethyl-2-imidazolidinone. One kind of these organic solvents may be used alone, or two or more kinds thereof may be used in combination.

Regarding a method for preparing the surface treatment liquid in the case of using a solubilizing agent and water, the solubilizing agent may be added after mixing the azole compound with water, the azole compound may be mixed with a mixed solution of water and the solubilizing agent, or water may be added after mixing the azole compound with the solubilizing agent.

The content of the solubilizing agent is preferably 0.1 wt% to 99 wt% in the surface treatment liquid, more preferably 0.5 wt% to 99 wt%, and still more preferably 1 wt% to 99 wt%.

The azole compound according to the present invention has an azole ring in the molecule, the azole ring interacts with a surface of the base material (metal, a resin material, and an inorganic material) to form a chemical bond, and therefore, the adhesion between different materials can be improved.

For example, when adhesion between copper and a resin material is taken as an example, the azole ring in the azole compound used in the implementation of the present invention interacts with the resin and copper to form a chemical bond, and a polymerizable group (-C₆H₄-CH=CH₂) interacts with the resin to form a chemical bond.

Therefore, when copper and the surface treatment liquid are brought into contact with each other, a chemical conversion film derived from the azole compound represented by the chemical formula (I) is formed on the surface of the copper by bonding to the azole ring. When a resin layer made of a resin material is formed on the surface of the chemical conversion film, the adhesion between the copper and the resin material can be enhanced as compared with the case where the resin layer is formed directly on the surface of the copper.

In the implementation of the present invention, the concentration of the azole compound represented by the chemical formula (I) in the surface treatment liquid is preferably 0.0001 mol/L to 1.0000 mol/L, more preferably 0.0010 mol/L to 0.5000 mol/L, and still more preferably 0.0100 mol/L to 0.1000 mol/L.

In order to improve stability of the surface treatment liquid and uniformity of the chemical conversion film, the surface treatment liquid may contain a substance that generates halide ions such as fluoride ions, chloride ions, bromide ions, and iodide ions or a substance that generates metal ions such as copper ions, iron ions, and zinc ions.

The halide ion exhibits an effect of uniformly forming a flat portion of the chemical conversion film. Examples of the substance that generates a halide ion include lithium fluoride, sodium fluoride, potassium fluoride, magnesium fluoride, calcium fluoride, lithium chloride, sodium chloride, potassium chloride, magnesium chloride, calcium chloride, lithium bromide, sodium bromide, potassium bromide, magnesium bromide, calcium bromide, lithium iodide, sodium iodide, potassium iodide, magnesium iodide, calcium iodide, ammonium fluoride, ammonium chloride, ammonium bromide, ammonium iodide, cuprous chloride, cupric chloride, cuprous bromide, and cupric bromide. The halogen compound may be contained as an impurity of other components.

The content of the halide ions in the surface treatment liquid is not particularly limited, and is, for example, preferably 0.10 mol/L or less (particularly 0 mol/L to 0.10 mol/L), more preferably 0.050 mol/L or less (particularly 0 mol/L to 0.050 mol/L), still more preferably 0.020 mol/L or less (particularly 0 mol/L to 0.020 mol/L), and particularly preferably 0.010 mol/L or less (particularly 0 mol/L to 0.010 mol/L).

The copper ion forms a complex with the azole compound, and can increase the strength of the chemical conversion film or the adhesive strength between the metal and the resin. The valence of the copper ion may be monovalent or divalent. Examples of the substance that generates copper ions include metal copper, copper sulfate (and a hydrate thereof (particularly pentahydrate)), copper formate (and a hydrate thereof (particularly tetrahydrate)), copper nitrate, cuprous chloride, cupric chloride, copper acetate (and a hydrate thereof (particularly monohydrate)), copper hydroxide, copper oxide, copper sulfide, copper carbonate, cuprous bromide, cupric bromide, copper phosphate, and copper benzoate.

The copper ions in the surface treatment liquid may contain copper ions eluted from metal copper or copper oxide contained in a copper circuit during the treatment of the copper circuit with the surface treatment liquid.

The content of copper ions in the surface treatment liquid is not particularly limited, and is, for example, preferably 1.00 mol/L or less (particularly 0 mol/L or more and 1.00 mol/L or less), more preferably 0.50 mol/L or less (particularly 0 mol/L or more and 0.50 mol/L or less), still more preferably 0.10 mol/L or less (particularly 0 mol/L or more and 0.10 mol/L or less), and particularly preferably 0.010 mol/L or less (particularly 0 mol/L or more and 0.010 mol/L or less).

Known coupling agents may be used in combination as long as the effects of the present invention are not impaired. Examples of known coupling agents include silane-based coupling agents (silane coupling agents) having a thiol group (mercapto group), a vinyl group, an epoxy group, a (meth)acrylic group, an amino group, a chloropropyl group, or the like.

Examples of such silane-based coupling agents include:
mercaptosilane compounds such as 3-mercaptopropyltrimethoxysilane and 3-mercaptopropylmethyldimethoxysilane;
vinylsilane compounds such as vinyltrichlorosilane, vinyltrimethoxysilane, and vinyltriethoxysilane;
styrylsilane compounds such as p-styryltrimethoxysilane;
epoxysilane compounds such as 2-(3,4-epoxycyclohexyl)ethyltrimethoxysilane, 3-glycidoxypropyltrimethoxysilane, 3-glycidoxypropylmethyldiethoxysilane, and 3-glycidoxypropyltriethoxysilane;
acryloxysilane compounds such as 3-acryloxypropyltrimethoxysilane;
methacryloxysilane compounds such as 3-methacryloxypropylmethyldimethoxysilane, 3-methacryloxypropyltrimethoxysilane, 3-methacryloxypropylmethyldiethoxysilane, and 3-methacryloxypropyltriethoxysilane;
aminosilane compounds such as N-2-(aminoethyl)-3-aminopropylmethyldimethoxysilane, N-2-(aminoethyl)-3-aminopropyltrimethoxysilane, N-2-(aminoethyl)-3-aminopropyltriethoxysilane, 3-aminopropyltrimethoxysilane, 3-aminopropyltriethoxysilane, 3-triethoxysilyl-N-(1,3-dimethylbutylidene)propylamine, N-phenyl-3-aminopropyltrimethoxysilane, and N-(vinylbenzyl)-2-aminoethyl-3-aminopropyltrimethoxysilane;
ureidosilane compounds such as 3-ureidopropyltriethoxysilane;
chloropropylsilane compounds such as 3-chloropropyltrimethoxysilane;
sulfide silane compounds such as bis(triethoxysilylpropyl)tetrasulfide; and
isocyanatosilane compounds such as 3-isocyanatopropyltriethoxysilane.

Other examples include aluminum-based coupling agents, titanium-based coupling agents, and zirconium-based coupling agents.

### (Treatment Method using Surface Treatment Liquid according to Present Invention)

A method of bringing the surface treatment liquid according to the present invention into contact with the surface of the base material is not particularly limited, and methods such as spraying, immersion, or coating can be adopted as in the case of the coupling agent described above.

The time (treatment time) for bringing the surface treatment liquid into contact with the base material is preferably 1 second to 10 minutes, and more preferably 5 seconds to 3 minutes. In the case where the treatment time is shorter than 1 second, the film thickness of the chemical conversion film formed on the base material surface becomes thin, and it is difficult to sufficiently obtain the adhesive force between materials of different properties. On the other hand, even in the case where the treatment time is longer than 10 minutes, there is no large difference in the film thickness of the chemical conversion film, and thus it is preferable to perform the treatment within 10 minutes from the viewpoint of productivity.

The temperature of the treatment liquid when the surface treatment liquid is brought into contact with the base material surface is preferably 5°C to 50°C, but may be appropriately set in relation to the treatment time.

After the surface treatment liquid according to the present invention is brought into contact with the base material, the base material may be washed with water and then dried, or may be dried without washing with water.

The drying is preferably performed at a temperature of room temperature to 150°C.

The water used for washing with water is preferably pure water such as ion exchange water or distilled water, but the method and time for washing with water are not particularly limited, and appropriate time and method such as spraying or immersion may be used.

The film thickness of the chemical conversion film is preferably 0.5 nm to 1,000 nm, more preferably 1 nm to 200 nm, and still more preferably 1 nm to 100 nm. In the case where the film thickness is 0.5 nm or more, adhesion between materials is sufficiently enhanced, and in the case where the film thickness is 1,000 nm or less, chemical resistance of the chemical conversion film can be maintained.

In the present invention, a surface of the chemical conversion film may be modified by subjecting the chemical conversion film after drying to a treatment such as plasma, laser, ion beam, ozone, heating, or humidification. Alternatively, the cleaning for the purpose of removing the resin and ion residues on the metal surface may be performed by using a processing method such as mechanical polishing using plasma, laser, ion beam, and pumice brush, or drilling.

When the surface of copper or a copper alloy (hereinafter, both may be simply referred to as copper) is treated, before the surface treatment liquid according to the present invention is brought into contact with the surface of copper, at least one pretreatment selected from pickling treatment, alkali treatment, roughening treatment, heat resistance treatment, rust prevention treatment, and chemical conversion treatment may be performed on the surface of copper.

The pickling treatment is performed to remove oil and fat components adhering to the surface of copper and to remove an oxide film on the surface of copper. For this pickling treatment, a solution such as a hydrochloric acid-based solution, a sulfuric acid-based solution, a nitric acid-based solution, a sulfuric acid-hydrogen peroxide-based solution, an organic acid-based solution, an inorganic acid-organic solvent-based solution, or an organic acid-organic solvent-based solution can be used.

The alkali treatment is performed to remove oil and fat components adhering to the surface of copper and to remove residues (for example, a dry film resist for forming a copper circuit) in the previous step. For the alkali treatment, a solution such as an aqueous solution or an organic solvent solution containing an alkali metal hydroxide such as sodium hydroxide and potassium hydroxide, an amine such as ammonia, ethanolamine, monopropanolamine, and tetramethylammonium hydroxide, sodium carbonate, sodium hydrogen carbonate, potassium carbonate, potassium hydrogen carbonate, ammonium carbonate, ammonium hydrogen carbonate, sodium acetate, potassium acetate, sodium phosphate, disodium hydrogen phosphate, potassium phosphate, or dipotassium hydrogen phosphate can be used.

The roughening treatment is performed to enhance adhesion between copper and a resin by an anchor effect, and an uneven shape is imparted to the surface of copper, so that the adhesion between copper and a resin material can be enhanced. In the roughening treatment, methods such as a micro-etching method, an electroplating method, an electroless plating method, an oxidation method (black oxide, brown oxide), an oxidation-reduction method, a brush-polishing method, and a jet-scrubbing method can be adopted.

In the micro-etching method, for example, an organic acid-cupric ion etching agent, a sulfuric acid-hydrogen peroxide etching agent, a persulfate etching agent, a copper chloride etching agent, or an iron chloride etching agent can be used. In the electroplating method, fine copper particles are deposited on the surface of copper to form unevenness on the surface of copper.

In the heat resistance treatment, a film of at least one selected from nickel, nickel-phosphorus, zinc, zinc-nickel, copper-zinc, copper-nickel, copper-nickel-cobalt, and nickel-cobalt is formed on the surface of copper. The film can be formed by adopting a known electroplating method, but the method is not limited to electroplating, and vapor deposition or other methods may be used.

The rust prevention treatment is performed to prevent oxidation corrosion of the surface of copper, and a method for forming a zinc or zinc alloy plating film, or an electrolytic chromate plating film on the surface of copper can be adopted. A treatment liquid containing an organic compound-based rust inhibitor such as a benzotriazole-based rust inhibitor may be brought into contact with the surface of copper.

In the chemical conversion treatment, a method for forming a tin passivation film or a method for forming a copper oxide passivation film can be adopted.

Before and/or after the surface treatment liquid according to the present invention is brought into contact with the surface of copper, an aqueous solution containing copper ions may be brought into contact with the surface of copper. The aqueous solution containing copper ions has a function of enhancing the film-forming property of the chemical conversion film formed on the surface of copper and a function of making the thickness of the chemical conversion film formed on the surface of copper uniform. The valence of the copper ion is not particularly limited, and the copper ion is a monovalent or divalent copper ion.

The copper ion source of the aqueous solution containing copper ions is not particularly limited as long as it is a copper salt soluble in water, and examples thereof include copper salts such as copper sulfate, copper nitrate, copper chloride, copper formate, and copper acetate. Ammonia or hydrochloric acid may be added to solubilize the copper salt in water.

Before and/or after the surface treatment liquid according to the present invention is brought into contact with the surface of copper, an acidic aqueous solution or an alkaline aqueous solution may be brought into contact with the surface of copper. The acidic aqueous solution or the alkaline aqueous solution also has a function of making the thickness of the chemical conversion film formed on the surface of copper uniform, similarly to the aqueous solution containing copper ions.

The acidic aqueous solution and the alkaline aqueous solution are not particularly limited, and examples of the acidic aqueous solution include aqueous solutions containing a mineral acid such as sulfuric acid, nitric acid, and hydrochloric acid, and an aqueous solution containing an organic acid such as formic acid, acetic acid, lactic acid, glycolic acid, and amino acids. Examples of the alkaline aqueous solution include aqueous solutions containing an alkali metal hydroxide such as sodium hydroxide and potassium hydroxide, an amine such as ammonia, ethanolamine, monopropanolamine, and tetramethylammonium hydroxide, sodium carbonate, sodium hydrogen carbonate, potassium carbonate, potassium hydrogen carbonate, ammonium carbonate, ammonium hydrogen carbonate, sodium acetate, potassium acetate, sodium phosphate, disodium hydrogen phosphate, potassium phosphate, or dipotassium hydrogen phosphate.

Before and/or after the surface treatment liquid according to the present invention is brought into contact with the surface of copper, an aqueous solution containing a known coupling agent may be brought into contact with the surface of copper.

Before and/or after the surface treatment agent according to the present invention is brought into contact with the surface of copper, a solution containing an azole compound may be brought into contact with the surface of copper. The solution containing an azole compound has a function of enhancing strength of the chemical conversion film formed on the surface of copper.

The azole compound is not particularly limited, and examples thereof include commercially available imidazole compounds, triazole compounds, and tetrazole compounds. Examples of the azole compounds include imidazole, 2-methylimidazole, 1,2,3-triazole, 1,2,4-triazole, 3-amino-1,2,4-triazole, 3-amino-5-methyl-1,2,4-triazole, 5-methyltetrazole, 5-aminotetrazole, and 5-phenyltetrazole.

The solution containing an azole compound contains water and/or a solubilizing agent in addition to the azole compound. Examples of the solubilizing agent include the solubilizing agents described above (acids, alkalis, and organic solvents).

The solution containing an azole compound may further contain an additive. Examples of the additive include a copper compound, a halogen compound, and other additives (for example, ammonium salts such as ammonium sulfate, ammonium formate, ammonium acetate, ammonium chloride, ammonium carbonate, and ammonium nitrate).

After the surface treatment liquid according to the present invention is brought into contact with the surface of copper, a treatment liquid containing a known organic compound-based rust inhibitor such as a benzotriazole-based rust inhibitor may be brought into contact therewith.

The surface treatment liquid according to the present invention can be used for treating a surface of at least one base material selected from the group consisting of a metal, an inorganic material, and a resin material, as described above. By treating a surface of the base material with the surface treatment liquid according to the present invention, a chemical conversion film can be formed on the surface of the base material, and adhesion to other materials can be enhanced.

In the present invention, two materials selected from the group consisting of a metal, an inorganic material, and a resin material, as described above, can adhere to each other by using the surface treatment liquid according to the present invention. The affinity between the two materials can be improved by adhering the two materials to each other through the layer of the chemical conversion film formed by the surface treatment liquid according to the present invention, and therefore, even materials of different properties can adhere to each other more firmly.

### (Adhesion Method Using Surface Treatment Liquid according to Present Invention)

The two materials can adhere to each other by a known method. For example, a method of bringing the surface treatment liquid according to the present invention into contact with a surface of a base material made of a metal, an inorganic material, or a resin material to form a chemical conversion film, and adhering another base material to a part of or the entire formed chemical conversion film by a method such as coating, pressure-bonding, and mixing, or by use of an adhesive or an adhesive sheet (film), or a combination of these methods can be adopted.

In addition, a method of bringing the surface treatment liquid according to the present invention into contact with surfaces of two base materials selected from a metal, an inorganic material, and a resin material to respectively form chemical conversion films on the surfaces of the two base materials, and adhering the two base materials to each other by a method such as coating, pressure-bonding, and mixing, or by use of an adhesive or an adhesive sheet (film), or a combination of these methods can be adopted.

The two materials, in particular, two materials with different properties can be adhered to each other as described above by using the surface treatment liquid according to the present invention, and therefore, the surface treatment liquid can be suitably used for various electric and electronic components, semiconductor wafers, and electronic devices such as printed wiring boards.

In the present invention, the surface treatment liquid according to the present invention can be suitably used for a base material formed of a metal, particularly copper or a copper alloy. For example, the surface treatment liquid is suitable for a surface treatment of copper or a copper alloy for the purpose of enhancing adhesion (adhesiveness) between a copper circuit (copper wiring layer) and a semi-cured or cured prepreg or solder resist, or semi-cured or cured dry film resist (insulating resin layer), and in a printed wiring board having an insulating resin layer in contact with the copper wiring layer, adhesion between the copper wiring layer and the insulating resin layer can be enhanced.

In an application example in a semiconductor wafer, the surface treatment liquid is suitable for a surface treatment of a semiconductor circuit for the purpose of enhancing adhesion (adhesiveness) between a semiconductor circuit formed on a semiconductor wafer and a protective film (for example, an insulating protective film such as a photosensitive positive-type buffer coat, a photosensitive negative-type buffer coat, a non-photosensitive buffer coat, or a bump protective film).

Further, in a package substrate (WL-CSP, FO-WLP, PLP), a 2.5-dimensional (2.5D) interposer substrate, or a three-dimensional (3D) interposer substrate in which a rewiring layer is formed on a semiconductor wafer, the surface treatment liquid is suitable for a surface treatment of a copper circuit rewiring layer for the purpose of enhancing adhesion (adhesiveness) between the copper circuit rewiring layer and an insulating material.

Examples of the protective film and the insulating material include a polyimide resin, a polybenzoxazole resin, and a silicone resin.

The printed wiring board can be produced by bringing a surface of a copper wire into contact with the surface treatment liquid according to the present invention, followed by washing with water and drying, and then forming an insulating resin layer on the surface of the copper wire. The method of this contact is as described above, and immersion of the copper wiring in the surface treatment liquid, spraying of the treatment liquid to the copper wire, or the like is simple and reliable and is preferable.

The method of washing with water is not particularly limited, and immersion of the copper wire in the washing water or spraying of the washing water to the surface of the copper wire is easy and reliable, and is preferable.

The insulating resin layer can be formed by a known method, for example, a method of attaching a semi-cured resin material or a method of applying a liquid resin material containing a solvent. Then, a via hole is formed to electrically connect the upper and lower wires. A multilayer printed wiring board can be produced by repeating this process.

In the method for forming a circuit of the printed wiring board, an example of a semi-additive method using the surface treatment liquid according to the present invention is described below.

A method for producing a circuit substrate includes at least one of:
(a) a step of preparing an insulating substrate or an insulating substrate having through-holes and via holes, the insulating substrate having a first conductive layer on a first surface, a second surface opposite to the first surface, and inner walls of the through-holes and the via holes;
(b) a step of forming a photo-crosslinkable resin layer and a mask layer on the first surface and the second surface, and covering the first conductive layer on the first surface, the second surface, and the inner walls of the through-holes and the via holes with the photo-crosslinkable resin layer and the mask layer;
(c) a step of pattern-exposing the photo-crosslinkable resin layer on the first surface, the second surface, and peripheries of the through-holes and the via holes;
(d) a step of removing the mask layer on the first surface, the second surface, and the peripheries of the through-holes and the via holes;
(e) a step of developing and removing uncured photo-crosslinkable resin layer on the first surface, the second surface, and the peripheries of the through-holes and the via holes by using a photo-crosslinkable resin layer-removing solution, to expose the first conductive layer on the first surface, the first conductive layer on the second surface, and the first conductive layer on the peripheries of the through-holes and the via holes;
(f) a step of forming a second conductive layer on the first conductive layer exposed to the first surface, the second surface, and the inner walls of the through-holes and the via holes by electrolytic plating;
(g) a step of removing the cured photo-crosslinkable resin layer on the first surface, the second surface, and the peripheries of the through-holes and the via holes to expose the first and second conductive layers on the first surface, the second surface, and the inner walls of the through-holes and the via holes;
(h) a step of removing the exposed first conductive layer by flash etching;
(i) a step of forming a third conductive layer on the first and second conductive layers of the first surface, the second surface and the inner walls of the through-holes and the via holes by electroless plating and electrolytic plating; and
(j) a step of laminating an insulating resin layer on the first, second, and third conductive layers on the first surface, the second surface, and the inner walls of the through-holes and the via holes,
and in this method, the surface treatment liquid according to the present invention is brought into contact with at least one metal layer or resist layer among the first, second, and third conductive layers on the first surface, the second surface and the inner walls of the through-holes and via holes, the insulating resin substrate, the photo-crosslinkable resin layer used for the etching resist layer or plating resist layer, and the insulating resin layer, to produce a printed wiring board.

Further, in the method for forming a circuit of the printed wiring board, an example of a subtractive construction method using the surface treatment liquid according to the present invention is described below.

A method for producing a circuit substrate includes at least one of:
(a) a step of preparing an insulating substrate or an insulating substrate having through-holes and via holes, the insulating substrate having a first conductive layer on a first surface, a second surface opposite to the first surface, and inner walls of the through-holes and the via holes;
(b) a step of forming a photo-crosslinkable resin layer and a mask layer on the first surface and the second surface, and covering the first conductive layer on the first surface, the second surface, and the inner walls of the through-holes and the via holes with the photo-crosslinkable resin layer and the mask layer;
(c) a step of pattern-exposing the photo-crosslinkable resin layer on the first surface, the second surface, and peripheries of the through-holes and the via holes;
(d) a step of removing the mask layer on the first surface, the second surface, and the peripheries of the through-holes and the via holes;
(e) a step of developing and removing uncured photo-crosslinkable resin layer on the first surface, the second surface, and the peripheries of the through-holes and the via holes by using a photo-crosslinkable resin layer-removing solution, to expose the first conductive layer on the first surface, the first conductive layer on the second surface, and the first conductive layer on the peripheries of the through-holes and the via holes;
(f) a step of removing the first conductive layer exposed to the first surface, the second surface, and the inner walls of the through-holes and the via holes by etching;
(g) a step of removing the cured photo-crosslinkable resin layer on the first surface, the second surface, and the peripheries of the through-holes and the via holes to expose the first and second conductive layers on the first surface, the second surface, and the inner walls of the through-holes and the via holes;
(h) a step of forming a third conductive layer on the first and second conductive layers of the first surface, the second surface and the inner walls of the through-holes and the via holes by electroless plating and electrolytic plating; and
(i) a step of laminating an insulating resin layer on the first, second, and third conductive layers on the first surface, the second surface, and the inner walls of the through-holes and the via holes,
and in this method, the surface treatment liquid according to the present invention is brought into contact with at least one metal layer or resist layer among the first, second, and third conductive layers on the first surface, the second surface and the inner walls of the through-holes and via holes, the insulating resin substrate, the photo-crosslinkable resin layer used for the etching resist layer or plating resist layer, and the insulating resin layer, to produce a printed wiring board.

The copper wire and the conductive layer may be produced by any method such as an electroless plating method, an electrolytic plating method, a vapor deposition method, a sputtering method, and a damascene method, and may include an inner via hole, a through hole, a connection terminal, and the like.

In addition, the "copper" according to the present invention is used in applications and forms of a foil (an electrolytic copper foil, a rolled copper foil, a resin-attached copper foil, a carrier-attached copper foil, an electroless copper foil, a sputtered copper foil, and a thin copper foil), a plating film (an electroless copper plating film and an electrolytic copper plating film), a thin film formed by a vapor deposition method, a sputtering method, a damascene method, and the like, a particle, a needle, a fiber, a wire, a rod, a tube, a plate, and the like used for an electronic device such as a printed wiring board and a lead frame, a decorative article, a building material, and the like. In the case of recent copper wires through which high-frequency electric signals flow, the surface of copper is preferably a smooth surface having an average roughness of 0.1 µm or less. The surface of copper may be plated with nickel, zinc, chromium, tin, or the like as a pretreatment.

Further, for example, in an application example in a lead frame during wire-bonding mounting, the surface treatment liquid according to the present invention is suitable for a surface treatment of a lead frame for the purpose of enhancing adhesion (adhesiveness) with a sealing resin or an adhesive during mounting of a semiconductor chip, in a metal surface in a lead frame-forming step, a frame metal surface after mounting a semiconductor chip (before and after a die-bonding and prebaking step), a frame metal surface after wire-bonding mounting, or a frame metal surface during the step up to resin-sealing (before and after a resin-molding and baking step).

Further, for example, in an application example in a lead frame during flip-chip mounting, the surface treatment liquid according to the present invention is suitable for a surface treatment of a lead frame for the purpose of enhancing adhesion (adhesiveness) with a sealing resin or an adhesive during mounting of a semiconductor chip, in a metal surface in a lead frame-forming step, a metal surface of a lead frame after a joining material (solder, Au-plating, Sn-plating, or the like) is temporarily placed, a metal surface of a frame after mounting a semiconductor chip (before or after an alignment step and a chip-mounting and baking step), a metal surface of a lead frame after main curing (before or after steps of reflow-heating, thermocompression bonding, ultrasonic, plasma, or the like), or a metal surface of a lead frame during the step up to resin-sealing.

Further, for example, in an application example in a fine wiring substrate in which an integration technology for closely disposing a semiconductor chip is enhanced, the surface treatment liquid according to the present invention is also suitable for a surface treatment of a copper circuit-wiring layer for the purpose of enhancing adhesion (adhesiveness) between the copper circuit-wiring layer and an insulating material, in a 2.5-dimensional (2.5D) organic substrate or a glass substrate, a component-embedded substrate (EPS substrate) in which a semiconductor is embedded in a substrate, or a coreless substrate.

Further, for example, the metal surface treatment liquid according to the present invention is suitable for a surface treatment of a copper circuit-wiring layer for the purpose of enhancing adhesion (adhesiveness) between the copper circuit-wiring layer and an insulating material in the case of performing via fill plating after embedding patterned wiring and processing upper and lower layers with a laser via, or in the case of using a circuit-embedded substrate (ETS substrate) with MIS structure, where conduction between the upper layer and the lower layer is achieved via copper pillars formed by plating and a mold resin is used in an insulating layer.

The carrier-attached copper foil treated with the surface treatment liquid according to the present invention refers to an ultrathin electrolytic copper foil used for a printed wiring board including a step of forming a circuit by any one of a semi-additive method, a subtractive method, a partly additive method, and a modified semi-additive method, and includes a copper foil carrier, a release layer laminated on the copper foil carrier, and an ultrathin copper layer laminated on the release layer. The surface of copper may be subjected to at least one pretreatment selected from the group consisting of a pickling treatment, a roughening treatment, a heat resistance treatment, a rust prevention treatment, and a chemical conversion treatment.

### (Resin Composition)

The azole compound according to the present invention can be used as a raw material for a resin composition.

The content of the azole compound according to the present invention in the resin composition is preferably 0.001 wt% to 100 wt%.

A cured product is obtained by polymerizing the azole compound according to the present invention, and a cured product obtained by copolymerizing the azole compound according to the present invention and another curable compound different from the azole compound according to the present invention can be obtained by allowing the other curable compound to coexist with the azole compound according to the present invention during the polymerization.

Examples of the other curable compound include an ene compound having a carbon-carbon double bond in the molecule (hereinafter, may be referred to as "first curable compound") and a compound having an epoxy group, an oxetane ring, or a vinyl group in the molecule (hereinafter, may be referred to as "second curable compound").

### (First Resin Composition)

The first resin composition contains the azole compound according to the present invention as an essential component, and optionally contains the first curable compound (an ene compound having a carbon-carbon double bond in the molecule).

The first curable compound includes both a polymerizable monomer and a polymerizable oligomer (semi-cured product) that has a structure in which the polymerizable monomers are partially polymerized.

Examples of the polymerizable monomer include:
(1) an alkyl (meth)acrylate monomer,
(2) a hydroxy group-containing monomer,
(3) a carboxyl group-containing monomer,
(4) an amino group-containing monomer,
(5) an acetoacetyl group-containing monomer,
(6) an isocyanate group-containing monomer,
(7) a glycidyl group-containing monomer,
(8) a monomer containing one aromatic ring,
(9) a monomer containing an alkoxy group and an oxyalkylene group,
(10) an alkoxyalkyl (meth)acrylamide monomer,
(11) a (meth)acrylamide monomer,
(12) a monofunctional unsaturated compound, and
(13) a polyfunctional unsaturated compound.

(1) Examples of the alkyl (meth)acrylate monomer include:
   methyl (meth)acrylate,
   ethyl (meth)acrylate,
   n-butyl (meth)acrylate,
   iso-butyl (meth)acrylate,
   tert-butyl (meth)acrylate,
   n-propyl (meth)acrylate,
   n-hexyl (meth)acrylate,
   2-ethylhexyl (meth)acrylate,
   n-octyl (meth)acrylate,
   isodecyl (meth)acrylate,
   lauryl (meth)acrylate,
   cetyl (meth)acrylate,
   stearyl (meth)acrylate,
   cyclohexyl (meth)acrylate,
   isobornyl (meth)acrylate, and
   polyisobornyl (meth)acrylate.
(2) Examples of the hydroxy group-containing monomer include:
   hydroxyalkyl (meth)acrylates such as 2-hydroxyethyl (meth)acrylate, 4-hydroxybutyl (meth)acrylate, 5-hydroxypentyl (meth)acrylate, 6-hydroxyhexyl (meth)acrylate, and 8-hydroxyoctyl (meth)acrylate;
   caprolactone-modified monomers such as caprolactone-modified 2-hydroxyethyl (meth)acrylate;
   oxyalkylene-modified monomers such as diethylene glycol (meth)acrylate and polyethylene glycol (meth)acrylate;
   primary hydroxy group-containing monomers such as 2-acryloyloxyethyl 2-hydroxyethyl phthalate, N-methylol (meth)acrylamide, and hydroxyethyl acrylamide;
   secondary hydroxy group-containing monomers such as 2-hydroxypropyl (meth)acrylate, 2-hydroxybutyl (meth)acrylate, 3-chloro-2-hydroxypropyl (meth)acrylate, propylene glycol diglycidyl ether-epoxy di(meth)acrylate, phenol glycidyl ether-epoxy (meth)acrylate, and bisphenol A diglycidyl ether-epoxy di(meth)acrylate; and
   tertiary hydroxy group-containing monomers such as 2,2-dimethyl 2-hydroxyethyl (meth)acrylate.
(3) Examples of the carboxyl group-containing monomer include (meth)acrylic acid, acrylic acid dimer, crotonic acid, maleic acid, maleic anhydride, fumaric acid, citraconic acid, glutaconic acid, itaconic acid, acrylamide N-glycolic acid, and cinnamic acid.
(4) Examples of the amino group-containing monomer include tert-butylaminoethyl (meth)acrylate, ethylaminoethyl (meth)acrylate, dimethylaminoethyl (meth)acrylate, and diethylaminoethyl (meth)acrylate.
(5) Examples of the acetoacetyl group-containing monomer include 2-(acetoacetoxy)ethyl (meth)acrylate and allyl acetoacetate.
(6) Examples of the isocyanate group-containing monomer include 2-acryloyloxyethyl isocyanate, 2-methacryloyloxyethyl isocyanate, and alkylene oxide adducts thereof.
(7) Examples of the glycidyl group-containing monomer include:
   glycidyl (meth)acrylate,
   epoxy (meth)acrylates, which are reaction products of epoxy compounds and (meth)acrylic acid, such as ethylene glycol diglycidyl ether-epoxy (meth) acrylate, resorcinol diglycidyl ether-epoxy (meth)acrylate, bis(4-hydroxyphenyl) sulfide diglycidyl ether-epoxy (meth)acrylate, phenol novolac type epoxy resin-(meth)acrylate, cresol novolac type epoxy resin-(meth)acrylate, bisphenol (e.g., bisphenol A, bisphenol F) type epoxy resin-(meth)acrylate, biphenol (e.g., 3,3',5,5'-tetramethylbiphenol) type epoxy resin-(meth)acrylate, and 1,3,5-tris(2,3-epoxypropyl)isocyanurate-(meth)acrylate, and
   glycidyl (meth)acrylates such as 4-hydroxybutyl (meth)acrylate glycidyl ether.
(8) Examples of the monomer containing one aromatic ring include:
   phenyl (meth)acrylate,
   benzyl (meth)acrylate,
   phenoxyethyl (meth)acrylate,
   phenoxydiethylene glycol (meth)acrylate,
   2-hydroxy-3-phenoxypropyl (meth)acrylate,
   styrene, and
   α-methylstyrene.
(9) Examples of the monomer containing an alkoxy group and an oxyalkylene group include:
   2-methoxyethyl (meth)acrylate,
   2-ethoxyethyl (meth)acrylate,
   3-methoxybutyl (meth)acrylate,
   2-butoxyethyl (meth)acrylate,
   2-butoxydiethylene glycol (meth)acrylate,
   methoxydiethylene glycol (meth)acrylate,
   methoxytriethylene glycol (meth)acrylate,
   ethoxydiethylene glycol (meth)acrylate,
   methoxydipropylene glycol (meth)acrylate,
   methoxypolyethylene glycol (meth)acrylate,
   octoxypolyethylene glycol-polypropylene glycol-mono(meth)acrylate,
   lauroxypolyethylene glycol mono(meth)acrylate, and
   stearoxypolyethylene glycol mono(meth)acrylate.
(10) Examples of the alkoxyalkyl (meth)acrylamide monomer include:
   methoxymethyl (meth)acrylamide,
   ethoxymethyl (meth)acrylamide,
   propoxymethyl (meth)acrylamide,
   isopropoxymethyl (meth)acrylamide,
   n-butoxymethyl (meth)acrylamide, and
   isobutoxymethyl (meth)acrylamide.
(11) Examples of the (meth)acrylamide monomer include:
   (meth)acryloylmorpholine,
   dimethyl(meth)acrylamide,
   diethyl(meth)acrylamide,
   (meth)acrylamide, and
   N-methylol(meth)acrylamide.
(12) Examples of the monofunctional unsaturated compound include biphenyl structure-containing (meth)acrylate-based compounds, and more specifically, examples thereof include:
   biphenyl (meth)acrylates such as o-biphenyl (meth)acrylate, m-biphenyl (meth)acrylate, and p-biphenyl (meth)acrylate;
   biphenyloxyalkyl (meth)acrylates such as o-biphenyloxymethyl (meth)acrylate, m-biphenyloxymethyl (meth)acrylate, p-biphenyloxymethyl (meth)acrylate, o-biphenyloxyethyl (meth)acrylate, m-biphenyloxyethyl (meth)acrylate, p-biphenyloxyethyl (meth)acrylate, o-biphenyloxypropyl (meth)acrylate, m-biphenyloxypropyl (meth)acrylate, and p-biphenyloxypropyl (meth)acrylate; and
   biphenyloxypolyalkylene glycol (meth)acrylates such as (o-biphenyloxy)diethylene glycol(meth) acrylate, (m-biphenyloxy)diethylene glycol (meth)acrylate, (p-biphenyloxy)diethylene glycol (meth)acrylate, (o-biphenyloxy)dipropylene glycol (meth)acrylate, (m-biphenyloxy)dipropylene glycol (meth)acrylate, (p-biphenyloxy)dipropylene glycol (meth)acrylate, (o-biphenyloxy)polyethylene glycol (meth)acrylate, (m-biphenyloxy)polyethylene glycol (meth)acrylate, (p-biphenyloxy)polyethylene glycol (meth)acrylate, (o-biphenyloxy)polypropylene glycol (meth)acrylate, (m-biphenyloxy)polypropylene glycol (meth)acrylate, and (p-biphenyloxy)polypropylene glycol (meth)acrylate.
(13) Examples of the polyfunctional unsaturated compound include bifunctional monomers, monomers with three or more functional groups, urethane (meth)acrylates, polyurethane (meth)acrylates, thiourethane (meth)acrylates, polythiourethane (meth)acrylates, the above-described epoxy (meth)acrylates, ester (meth)acrylates, polyester (meth)acrylates, and polyether (meth)acrylates.

Specific examples of the bifunctional monomer include:
ethylene glycol di(meth)acrylate,
diethylene glycol di(meth)acrylate,
triethylene glycol di(meth)acrylate,
tetraethylene glycol di(meth)acrylate,
polyethylene glycol di(meth)acrylate,
propylene glycol di(meth)acrylate,
dipropylene glycol di(meth)acrylate,
tripropylene glycol di(meth)acrylate,
polypropylene glycol di(meth)acrylate,
butylene glycol di(meth)acrylate,
neopentyl glycol di(meth)acrylate,
ethylene oxide-modified bisphenol A type di(meth)acrylate,
propylene oxide-modified bisphenol A type di(meth)acrylate,
1,6-hexanediol di(meth)acrylate,
1,6-hexanediol ethylene oxide-modified di(meth)acrylate,
glycerin di(meth)acrylate,
pentaerythritol di(meth)acrylate,
ethylene glycol diglycidyl ether di(meth)acrylate,
diethylene glycol diglycidyl ether di(meth)acrylate,
phthalic acid diglycidyl ester di(meth)acrylate,
hydroxypivalic acid-modified neopentyl glycol di(meth)acrylate,
isocyanuric acid ethylene oxide-modified diacrylate, and
2-(meth)acryloyloxyethyl acid phosphate diester.

Specific examples of the monomers with three or more functional groups include:
trimethylolpropane tri(meth)acrylate,
ditrimethylolpropane tetra(meth)acrylate,
pentaerythritol tri(meth)acrylate,
pentaerythritol tetra(meth)acrylate,
dipentaerythritol tri(meth)acrylate,
dipentaerythritol tetra(meth)acrylate,
dipentaerythritol penta(meth)acrylate,
dipentaerythritol hexa(meth)acrylate,
tri(meth)acryloyloxyethoxytrimethylolpropane,
glycerin polyglycidyl ether poly(meth)acrylate,
tris(2-(meth)acryloyloxyethyl)isocyanurate,
isocyanuric acid ethylene oxide-modified tri(meth)acrylate,
ethylene oxide-modified dipentaerythritol penta (meth) acrylate,
ethylene oxide-modified dipentaerythritol hexa(meth)acrylate,
ethylene oxide-modified pentaerythritol tri(meth)acrylate,
ethylene oxide-modified pentaerythritol tetra(meth)acrylate, and
succinic acid-modified pentaerythritol tri(meth)acrylate.

In addition to the polymerizable monomers described above, examples thereof include:
divinylbenzene, piperylene, isoprene, pentadiene, vinylcyclohexene, chloroprene, butadiene, methylbutadiene, cyclopentadiene, methylpentadiene, acrylonitrile, methacrylonitrile, vinyl acetate, vinyl propionate, vinyl stearate, vinyl chloride, vinylidene chloride, alkyl vinyl ether, vinyl toluene, vinylpyridine, vinylpyrrolidone, dialkyl itaconate, dialkyl fumarate, allyl alcohol, acryloyl chloride, methyl vinyl ketone, N-acrylamidomethyltrimethylammonium chloride, allyltrimethylammonium chloride, dimethylallyl vinyl ketone, 2-chloroethyl vinyl ether, triallyl isocyanurate, tetraallyl glycoluril,
N-vinylpyrrolidone, N-vinylcaprolactam, ethylene glycol diallyl carbonate, trimellitic acid triallyl ester,
trifluoroethyl (meth)acrylate,
tribromobenzyl (meth)acrylate,
perfluorooctylethyl (meth)acrylate,
sulfur-containing (meth)acrylate,
sulfur-containing polyfunctional (meth)acrylate, and
(meth)acryloyloxypropyltris(methoxy)silane.

The first resin composition contains the azole compound according to the present invention as an essential component and optionally contains the first curable compound described above. As the first curable compound, the polymerizable monomer and the polymerizable oligomer described above may be used in combination. As the polymerizable monomer, the polymerizable monomers exemplified above may be used in combination (different types of polymerizable monomers may be used in combination). As the polymerizable oligomer, different types of polymerizable oligomers may be used in combination.

Regarding the content ratio of each of the azole compound according to the present invention and the first curable compound in the first resin composition, the content ratio is preferably an appropriate ratio in a range where the content of the first curable compound is 0 to 1000 times (by weight) with respect to the content of the azole compound according to the present invention, and is more preferably an appropriate ratio within a range where the content of the first curable compound is 0.01 times to 100 times (by weight) with respect to the content of the azole compound according to the present invention.

The first resin composition may contain a thiol compound as a curing agent. Examples of the thiol compound include:
aliphatic thiol compounds such as ethanedithiol, propanedithiol, hexamethylenedithiol, decamethylenedithiol, tolylene-2,4-dithiol, 2,2-bis(mercaptomethyl)-1,3-propanedithiol, 2-(mercaptomethyl)-2-methyl-1,3-propanedithiol, and 2-ethyl-2-(mercaptomethyl)-1,3-propanedithiol;
aromatic thiol compounds such as benzenedithiol, toluenedithiol, and xylenedithiol (p-xylenedithiol);
cyclic sulfide compounds such as 1,4-dithiane ring-containing polythiol compounds represented by the chemical formula (VI);
mercaptoalkyl sulfide compounds such as 3-thiapentane-1,5-dithiol and 4-mercaptomethyl-3,6-dithia-1,8-octanedithiol;
mercaptopropionic acid esters such as pentaerythritol tetrakis(3-mercaptopropionate);
epoxy resin-terminated mercapto compounds;
mercaptoalkyl ether compounds such as 3,6-dioxa-1,8-octanedithiol, mercaptoalkyl ether disulfide compounds represented by the chemical formula (VII), 2,2'-[[2,2-bis[(2-mercaptoethoxy)methyl]-1,3-propanediyl]bis(oxy)]bisethanethiol, 3,3'-[[2,2-bis[(3-mercaptopropoxy)methyl]-1,3-propanediyl]bis(oxy)]bis-1-propanethiol, 3-[2,2-bis[(3-mercaptopropoxy)methyl]butoxy]-1-propanethiol, 3-(3-mercaptopropoxy)-2,2-bis[(3-mercaptopropoxy)methyl]-1-propanol, and 2,2-bis[(3-mercaptopropoxy)methyl]-1-butanol;
1,3,4,6-tetrakis(2-mercaptoethyl)glycoluril, and 1,3,4,6-tetrakis(3-mercaptopropyl)glycoluril, and these may be used in combination.

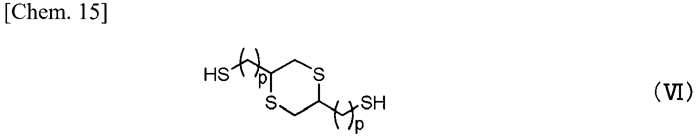

(In the formula (VI), p's are the same as or different from each other, and represent an integer of 1 to 5.)

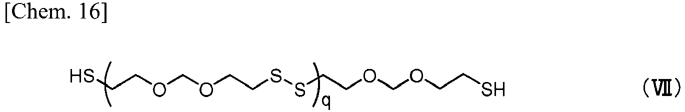

(In the formula (VII), q represents an integer of 1 to 20.)

In the first resin composition, the content of the thiol compound is preferably 0.1 parts by weight to 100 parts by weight, and more preferably 0.5 parts by weight to 20 parts by weight with respect to 100 parts by weight of the curable compound (total of the azole compound according to the present invention and the first curable compound).

Examples of a method of polymerizing (curing) the first resin composition include methods of performing photocuring and thermal curing.

Examples of the photocuring method include a method of radiating active energy rays, and preferably a method of using a photopolymerization initiator in combination. Examples of the active energy ray include light, radiation, electromagnetic waves, and electron beams, and electron beams or light in an ultraviolet to infrared wavelength range is preferable. As the light source, for example, an ultra-high pressure mercury light source or a metal halide light source can be used in the case of irradiation with ultraviolet rays, a metal halide light source or a halogen light source can be used in the case of irradiation with visible rays, and a halogen light source can be used in the case of irradiation with infrared rays. In addition, a light source such as a laser or an LED corresponding to light emission of various wavelengths, which has been widely used in recent years, may be used.

The irradiation amount of the active energy rays can be appropriately set according to the type of the light source or the like.

The photopolymerization initiator can be selected from a photoradical polymerization initiator and a photoanion polymerization initiator, and both may be contained in the resin composition. In photocuring, thermal polymerization (thermal curing) methods may be used in combination in order to enhance production efficiency and properties of the cured product.

The photoradical polymerization initiator is not particularly limited as long as it is a commonly used photoradical polymerization initiator, and examples thereof include:
acetophenones such as acetophenone, diethoxyacetophenone, 2,2-dimethoxy-2-phenylacetophenone, 2,2-diethoxy-2-phenylacetophenone, 1,1-dichloroacetophenone, 2-hydroxy-2-methyl-1-phenylpropan-1-one, acetophenone dimethyl ketal, benzyl dimethyl ketal, 4-(2-hydroxyethoxy)phenyl-(2-hydroxy-2-propyl)ketone, 1-hydroxycyclohexyl phenyl ketone, 2-methyl-2-morpholino(4-thiomethylphenyl)propan-1-one, 2-benzyl-2-dimethylamino-1-(4-morpholinophenyl)butanone, and 2-hydroxy-2-methyl-1-[4-(1-methylvinyl)phenyl]propanone oligomer;
benzoins such as benzoin, benzoin methyl ether, benzoin ethyl ether, benzoin propyl ether, benzoin isopropyl ether, and benzoin isobutyl ether;
benzophenones such as benzophenone, hydroxybenzophenone, o-benzoyl methyl benzoate, 4-phenylbenzophenone, 4-benzoyl-4'-methyl-diphenyl sulfide, 4,4'-bis(methylamino)benzophenone, 3,3',4,4'-tetra(t-butylperoxycarbonyl)benzophenone, 2,4,6-trimethylbenzophenone, 4-benzoyl-N,N-dimethyl-N-[2-(1-oxo-2-propenyloxy)ethyl]benzenemethanaminium bromide, and (4-benzoylbenzyl)trimethylammonium chloride;
thioxanthones such as 2-isopropylthioxanthone, 4-isopropylthioxanthone, 2,4-diethylthioxanthone, 2,4-dichlorothioxanthone, 1-chloro-4-propoxythioxanthone, and 2-(3-dimethylamino-2-hydroxy)-3,4-dimethyl-9H-thioxanthone-9-one mesochloride;
acylphosphine oxides such as 2,4,6-trimethylbenzoyl-diphenylphosphine oxide, bis(2,6-dimethoxybenzoyl)-2,4,4-trimethyl-pentylphosphine oxide, and bis(2,4,6-trimethylbenzoyl)-phenylphosphine oxide; and
methylphenylglyoxylate. These may be used alone or in combination of two or more kinds thereof.

The photoanionic polymerization initiator is not particularly limited as long as it is commonly used, and examples thereof include onium salts and carbamates.

Examples of the onium salts include 1,2-diisopropyl-3-(bis(dimethylamino)methylene) guanidium 2-(3-benzoylphenyl)propionate and 1,2-dicyclohexyl-4,4,5,5-tetramethylbiguanidium n-butyltriphenylborate. Examples of the carbamates include 2-nitrophenylmethylpiperidine-1-carboxylate, 1-(anthraquinone-2-yl)ethyl imidazole carboxylate, 1-(3-(2-hydroxyphenyl)-2-propenoyl)piperidine, and 9-anthranylmethyldiethylcarbamate.

In the first resin composition, the content of the photopolymerization initiator is preferably 0.1 parts by weight to 20 parts by weight, more preferably 0.2 parts by weight to 15 parts by weight, and still more preferably 0.5 parts by weight to 10 parts by weight with respect to 100 parts by weight of the curable compound (total of the azole compound according to the present invention and the first curable compound).

The first resin composition may contain an auxiliary agent for a photoradical polymerization initiator. Examples of the auxiliary agent include triethanolamine, triisopropanolamine, 4,4'-dimethylaminobenzophenone (Michler's ketone), 4,4'-diethylaminobenzophenone, 2-dimethylaminoethylbenzoic acid, ethyl 4-dimethylaminobenzoate, (n-butoxy)ethyl 4-dimethylaminobenzoate, isoamyl 4-dimethylaminobenzoate, 2-ethylhexyl 4-dimethylaminobenzoate, 2,4-diethylthioxanthone, and 2,4-diisopropylthioxanthone.

In addition, the first resin composition may contain a sensitizer in order to expand the photosensitive wavelength region and increase the sensitivity. Examples of the sensitizer include benzophenone, p,p'-tetramethyldiaminobenzophenone, p,p'-tetraethylaminobenzophenone, 2-chlorothioxanthone, anthrone, 9-ethoxyanthracene, anthracene, pyrene, perylene, phenothiazine, thioxanthone, benzyl, acridine orange, benzoflavin, setoflavin-T, 9,10-diphenylanthracene, 9-fluorenone, acetophenone, phenanthrene, 2-nitrofluorene, 5-nitroacenaphthene, benzoquinone, 2-chloro-4-nitroaniline, N-acetyl-p-nitroaniline, p-nitroaniline, N-acetyl-4-nitro-1-naphthylamine, picramide, anthraquinone, 2-ethylanthraquinone, 2-tert-butylanthraquinone, 1,2-benzanthraquinone, 3-methyl-1,3-diaza-1,9-benzanthrone, dibenzalacetone, 1,2-naphthoquinone, 3,3'-carbonyl-bis(5,7-dimethoxycarbonylcoumarin), and coronene.

The content of the sensitizer in the first resin composition is, for example, preferably 0.1 parts by weight to 20 parts by weight, more preferably 0.2 parts by weight to 15 parts by weight, and still more preferably 0.5 parts by weight to 10 parts by weight with respect to 100 parts by weight of the curable compound (total of the azole compound according to the present invention and the first curable compound).

On the other hand, examples of a method of thermally curing the first resin composition include a method using a thermal polymerization initiator in combination. The thermal polymerization initiator can be selected from a thermal radical polymerization initiator and a thermal anionic polymerization initiator, and both may be contained in the resin composition.

Regarding conditions of the thermal curing, the heating temperature and the heating time can be appropriately set, but are preferably set in a range of 60°C to 130°C for 30 minutes to 240 minutes, and more preferably set in a range of 70°C to 125°C for 30 minutes to 120 minutes.

Examples of the heating method include hot air circulation, infrared heating, and high-frequency heating. As the curing device, a closed curing furnace, a tunnel furnace capable of performing continuous curing, or the like can be used.

The thermal radical polymerization initiator is not particularly limited as long as it is commonly used. Examples thereof include:
peroxides such as diisopropyl peroxydicarbonate, benzoyl peroxide, t-butyl peroxyisobutyrate, t-hexyl peroxyisopropyl monocarbonate, t-hexylperoxy-2-ethylhexanoate, 1,1,3,3-tetramethylbutylperoxy-2-ethylhexanoate, t-butyl peroxypivalate, t-hexyl peroxypivalate, t-butyl peroxyneodecanoate, t-hexyl peroxyneodecanoate, 1,1,3,3-tetramethylbutyl peroxyneodecanoate, 1,1-bis(t-hexylperoxy) cyclohexane, benzoyl peroxide, 3,5,5-trimethylhexanoyl peroxide, and lauroyl peroxide; and
azo compounds such as azobisisobutyronitrile, 2,2'-azobis(2-methylbutyronitrile), and dimethyl 2,2'-azobis(2-methylpropionate).

These may be used in combination.

The thermal anionic polymerization initiator is not particularly limited as long as it is commonly used. Examples thereof include amines and imidazoles, and these may be used in combination.

Examples of the amines include diethylenetriamine, triethylenetetramine, isophoronediamine, xylylenediamine, diaminodiphenylmethane, and 1,3,4,6-tetrakis(3-aminopropyl)glycoluril. Examples of the imidazoles include 2-methylimidazole, 2-ethyl-4-methylimidazole, and 2-phenylimidazole.

The content of the thermal polymerization initiator in the first resin composition is preferably 0.001 wt% to 20 wt%, and more preferably 0.01 wt% to 10 wt%, with respect to the entire resin composition (total amount).

In the case where the first resin composition contains an epoxy resin (epoxy compound) as an additive (modifier), a photocationic polymerization initiator or a thermal cationic polymerization initiator may be used.

The photocationic polymerization initiator is not particularly limited as long as it is commonly used, and examples thereof include onium salts and organometallic complexes.

Examples of the onium salts include diazonium salts, sulfonium salts, and iodonium salts. Examples of the organometallic complexes include iron-allene complexes, titanocene complexes, and arylsilanol-aluminum complexes.

Examples of commercially available photocationic polymerization initiators include "Adeka Optomer SP-150 (trade name)" and "Adeka Optomer SP-170 (trade name)" manufactured by Adeka Corporation, "UVE-1014 (trade name)" manufactured by General Electric Company, "CD-1012 (trade name)" manufactured by Sartomer Corporation, and "CPI-100P (trade name)" manufactured by San-Apro Ltd..

Examples of a counter anion of the photocationic polymerization initiator include SbF₆⁻, AsF₆⁻, B(C₆F₅)₄⁻, and PF₆⁻.

The thermal cationic polymerization initiator is not particularly limited as long as it is commonly used, and examples thereof include various onium salts such as quaternary ammonium salts, phosphonium salts, and sulfonium salts, and organometallic complexes, and these may be used in combination.

Examples of commercially available onium salts include "Adeka Opton CP-66 (trade name)" and "Adeka Opton CP-77 (trade name)" manufactured by Adeka Corporation, "San-Aid SI-60L (trade name)", "San-Aid SI-80L (trade name)" and "San-Aid SI-100L (trade name)" manufactured by Sanshin Chemical Industry Co., Ltd., and "CI series (trade name)" manufactured by Nippon Soda Co., Ltd..

Examples of the organometallic complexes include alkoxysilane-aluminum complexes.

The first resin composition may further contain, as long as the effects of the present invention are not impaired,
a pigment (titanium white, cyanine blue, watching red, red iron oxide, carbon black, aniline black, manganese blue, iron black, ultramarine blue, Hansa red, Chrome yellow, Chrome green, and the like),
an inorganic filler (calcium carbonate, kaolin, clay, talc, mica, barium sulfate, lithopone, gypsum, zinc stearate, pearlite, quartz, quartz glass, silica powder such as fused silica and spherical silica, an oxide such as spherical alumina, crushed alumina, magnesium oxide, beryllium oxide, titanium oxide, and zirconium oxide, a nitride such as boron nitride, silicon nitride, and aluminum nitride, a carbide such as silicon carbide, a hydroxide such as aluminum hydroxide and magnesium hydroxide, a metal such as copper, silver, iron, aluminum, nickel, and titanium, an alloy, a carbon-based material such as diamond and carbon, and the like),
a thermoplastic resin and a thermosetting resin (a homopolymer such as high-density, medium-density or low-density polyethylene, polypropylene, polybutene, and polypentene, an ethylenepropylene copolymer, a polyamide resin such as nylon-6 and nylon-6,6, a vinyl chloride resin, a nitrocellulose resin, a vinylidene chloride resin, an acrylic resin (including curable compounds other than the polymerizable monomers described above), an acrylamide resin, a styrene resin, a vinyl ester resin, a polyester resin, a phenolic resin (phenolic compound), an epoxy resin (epoxy compound), a silicone resin, a fluorine resin, an elastomer resin such as acrylic rubber and urethane rubber, a graft copolymer such as a methyl methacrylate-butadiene-styrene graft copolymer and an acrylonitrile-butadiene-styrene graft copolymer, and the like),
a reinforcing agent (glass fiber, carbon fiber, and the like),
an anti-sagging agent (hydrogenated castor oil, fine particle silica anhydride, and the like),
a matting agent (fine powder silica, paraffin wax, and the like),
a grinding agent (zinc stearate, and the like),
an internal release agent (a fatty acid such as stearic acid, a fatty acid metal salt such as calcium stearate, a fatty acid amide such as stearic acid amide, a fatty acid ester, polyolefin wax, paraffin wax, and the like),
a diluent (an organic solvent such as n-butyl alcohol, methyl ethyl ketone (MEK), propylene glycol monomethyl ether acetate (PGMEA), and toluene, water, and a mixture of an organic solvent and water),
a coupling agent (a silane coupling agent such as N-(2-aminoethyl)-3-aminopropyltrimethoxysilane and 3-glycidoxypropyltrimethoxysilane, and the like),
a chain transfer agent (a thiol compound such as pentaerythritol tetrakis(3-mercaptopropionate), and the like), and
an additive (a modifier) such as a surfactant, a leveling agent, a defoamer, a fragrance, a flame retardant, and a dye.

The thermoplastic resin and the thermosetting resin include a resin (compound) having a cardo structure (a skeleton structure in which four aromatic rings are bonded to a carbon atom) represented by the chemical formula (VIII).

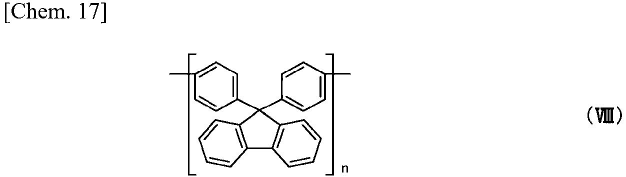

(In the formula (VIII), n represents a polymerization degree.)

Examples of the compound having a cardo structure include monomers such as 9,9-bis(4-glycidyloxyphenyl)fluorene, 9,9-bis(4-hydroxyphenyl)fluorene, 9,9-bis(4-hydroxy-3-methylphenyl)fluorene, 9,9-bis[4-(2-(meth)acryloyloxyethoxy)phenyl]fluorene, 9,9-bis[4-(3-(meth)acryloyloxy-2-hydroxypropoxy)phenyl] fluorene, 9,9-bis(cyanomethyl)fluorene, and 9,9-bis(3-aminopropyl)fluorene.

In the first resin composition, the content of the additive (modifier) may be 0.01 wt% to 50 wt% based on the entire resin composition (total amount).

A method for preparing the first resin composition is not particularly limited, and for example, the first resin composition can be prepared by mixing the azole compound according to the present invention with the first curable compound, the photopolymerization initiator and/or the thermal polymerization initiator, and the additive (modifier), or by mixing a solution obtained by dissolving or dispersing the naphthalene compound according to the present invention in a diluent (organic solvent) with the first curable compound, the photopolymerization initiator and/or the thermal polymerization initiator, and the additive (modifier).

As a mixing method, a known method can be adopted.

### (Second Resin Composition)

A second resin composition contains the azole compound according to the present invention as an essential component, and optionally contains a second curable compound (a compound having an epoxy group, an oxetane ring, or a vinyl group in the molecule).

The second curable compound includes both a polymerizable monomer and a polymerizable oligomer (semi-cured product) that has a structure in which the polymerizable monomers are partially polymerized.

Examples of the polymerizable monomer include known epoxy compounds (note: may be referred to as epoxy resins), oxetane compounds, epoxy-oxetane compounds (having an oxirane ring and an oxetane ring in the molecule), and vinyl monomers.

The epoxy compound is not particularly limited as long as the epoxy compound has an oxirane ring (epoxy group/glycidyl group) in the molecule, and examples thereof include:
polyglycidyl ethers obtained by reacting epichlorohydrin and polyhydric phenols such as bisphenol A, bisphenol F, bisphenol AD, catechol, and resorcinol, or polyhydric alcohols such as glycerin and polyethylene glycol;
glycidyl ether esters obtained by reacting epichlorohydrin and a hydroxycarboxylic acid such as p-hydroxybenzoic acid and β-hydroxynaphthoic acid;
polyglycidyl esters obtained by reacting epichlorohydrin and a polycarboxylic acid such as phthalic acid and terephthalic acid;
glycidyl glycoluril compounds having two or more epoxy groups in the molecule, such as 1,3,4,6-tetraglycidyl glycoluril;
alicyclic epoxy compounds such as 3',4'-epoxycyclohexylmethyl 3,4-epoxycyclohexane carboxylate;
nitrogen-containing cyclic epoxy compounds such as triglycidyl isocyanurate and hydantoin type epoxy compounds;
epoxidized phenol novolac resins, epoxidized cresol novolac resins, epoxidized polyolefins, cyclic aliphatic epoxy resins, and urethane-modified epoxy resins; and
epoxy-modified organopolysiloxane compounds obtained by a hydrosilylation addition reaction between an organic compound having a carbon-carbon double bond and a glycidyl group and a silicon compound having a SiH group (for example, an epoxy-modified organopolysiloxane compound disclosed in JP 2004-99751A or JP 2006-282988A). These may be used in combination.

The oxetane compound is not particularly limited as long as it has an oxetane ring (oxetanyl group/oxetane group) in the molecule, and examples thereof include:
3-ethyl-3-hydroxymethyloxetane,
3-(meth)allyloxymethyl-3-ethyloxetane,
(3-ethyl-3-oxetanylmethoxy)methylbenzene,
4-fluoro-[1-(3-ethyl-3-oxetanylmethoxy)methyl]benzene,
4-methoxy-[1-(3-ethyl-3-oxetanylmethoxy)methyl]benzene,
[1-(3-ethyl-3-oxetanylmethoxy)ethyl]phenyl ether,
isobutoxymethyl (3-ethyl-3-oxetanylmethyl)ether,
isobornyl oxyethyl (3-ethyl-3-oxetanylmethyl)ether,
isobornyl (3-ethyl-3-oxetanylmethyl)ether,
2-ethylhexyl (3-ethyl-3-oxetanylmethyl)ether,
ethyldiethylene glycol (3-ethyl-3-oxetanylmethyl)ether,
dicyclopentadiene (3-ethyl-3-oxetanylmethyl)ether,
dicyclopentenyloxyethyl (3-ethyl-3-oxetanylmethyl)ether,
dicyclopentenyl (3-ethyl-3-oxetanylmethyl)ether,
tetrahydrofurfuryl (3-ethyl-3-oxetanylmethyl)ether,
2-hydroxyethyl (3-ethyl-3-oxetanylmethyl)ether,
2-hydroxypropyl (3-ethyl-3-oxetanylmethyl)ether,
butoxyethyl (3-ethyl-3-oxetanylmethyl)ether,
bornyl (3-ethyl-3-oxetanylmethyl)ether,
3,7-bis(3-oxetanyl)-5-oxa-nonane,
3,3'-(1,3-(2-methylenyl)propanediylbis(oxymethylene))bis-(3-ethyloxetane),
1,4-bis[(3-ethyl-3-oxetanylmethoxy)methyl]benzene,
1,2-bis[(3-ethyl-3-oxetanylmethoxy)methyl]ethane,
1,3-bis[(3-ethyl-3-oxetanylmethoxy)methyl]propane,
ethylene glycol bis(3-ethyl-3-oxetanylmethyl)ether,
dicyclopentenylbis(3-ethyl-3-oxetanylmethyl)ether,
triethylene glycol bis(3-ethyl-3-oxetanylmethyl)ether,
tetraethylene glycol bis(3-ethyl-3-oxetanylmethyl)ether,
tricyclodecanediyldimethylene(3-ethyl-3-oxetanylmethyl)ether,
trimethylolpropane tris(3-ethyl-3-oxetanylmethyl)ether,
1,4-bis(3-ethyl-3-oxetanylmethoxy)butane,
1,6-bis(3-ethyl-3-oxetanylmethoxy)hexane,
pentaerythritol tris(3-ethyl-3-oxetanylmethyl)ether,
pentaerythritol tetrakis(3-ethyl-3-oxetanylmethyl)ether,
polyethylene glycol bis(3-ethyl-3-oxetanylmethyl)ether,
dipentaerythritol hexakis(3-ethyl-3-oxetanylmethyl)ether,
dipentaerythritol pentakis(3-ethyl-3-oxetanylmethyl)ether,
dipentaerythritol tetrakis(3-ethyl-3-oxetanylmethyl)ether,
caprolactone-modified dipentaerythritol hexakis(3-ethyl-3-oxetanylmethyl)ether,
caprolactone-modified dipentaerythritol pentakis(3-ethyl-3-oxetanylmethyl)ether,
ditrimethylolpropane tetrakis(3-ethyl-3-oxetanylmethyl)ether,
EO-modified bisphenol A bis(3-ethyl-3-oxetanylmethyl)ether,
PO-modified bisphenol A bis(3-ethyl-3-oxetanylmethyl)ether,
EO-modified hydrogenated bisphenol A bis(3-ethyl-3-oxetanylmethyl) ether,
PO-modified hydrogenated bisphenol A bis(3-ethyl-3-oxetanylmethyl)ether, and
EO-modified bisphenol F (3-ethyl-3-oxetanylmethyl)ether.

The epoxy-oxetane compound is not particularly limited as long as it has an oxirane ring (same as above) and an oxetane ring (same as above) in the molecule, and examples thereof include the compounds described in US 3,457,193B, JP 2005-002191A, JP 2007-270070A, JP 2010-111713A, and JP 2011-208089A. The epoxy oxetane compounds described in these literatures are incorporated herein by reference.

The vinyl monomer is not particularly limited as long as it is a cationically polymerizable vinyl monomer, and examples thereof include styrenes, alkenyl ethers, indene, and N-vinylcarbazole. Styrenes and alkenyl ethers are preferable.

Examples of the styrenes include styrene, o-methylstyrene, m-methylstyrene, p-methylstyrene, o-methoxystyrene, m-methoxystyrene, p-methoxystyrene, o-chlorostyrene, m-chlorostyrene, and p-chlorostyrene.

Examples of the alkenyl ethers include alkyl vinyl ethers such as methyl vinyl ether, ethyl vinyl ether, propyl vinyl ether, isopropyl vinyl ether, n-butyl vinyl ether, sec-butyl vinyl ether, tert-butyl vinyl ether, isobutyl vinyl ether, n-amyl vinyl ether, and isoamyl vinyl ether; fluoroalkyl vinyl ethers such as trifluoromethyl vinyl ether, pentafluoroethyl vinyl ether, and 2,2,2-trifluoroethyl vinyl ether; alkoxyalkyl vinyl ethers such as 2-methoxyethyl vinyl ether, 2-ethoxyethyl vinyl ether, 2-tetrahydropyranyl vinyl ether, and 2-tetrahydrofuranyl vinyl ether; cycloalkyl vinyl ethers such as cyclopentyl vinyl ether, cyclohexyl vinyl ether, cycloheptyl vinyl ether, cyclooctyl vinyl ether, 2-bicyclo[2.2.1]heptyl vinyl ether, 2-bicyclo[2.2.2]octyl vinyl ether, 8-tricyclo[5.2.1.0^{2,6} ]decanyl vinyl ether, 1-adamantyl vinyl ether, and 2-adamantyl vinyl ether; aryl vinyl ethers such as phenyl vinyl ether, 4-methylphenyl vinyl ether, 4-trifluoromethylphenyl vinyl ether, and 4-fluorophenyl vinyl ether; and aryl alkyl vinyl ethers such as benzyl vinyl ether and 4-fluorobenzyl vinyl ether.

The second resin composition contains the azole compound according to the present invention as an essential component and optionally contains the second curable compound described above. As the second curable compound, the polymerizable monomer and the polymerizable oligomer described above may be used in combination. As the polymerizable monomer, the polymerizable monomers exemplified above may be used in combination (different types of polymerizable monomers may be used in combination). As the polymerizable oligomer, different types of polymerizable oligomers may be used in combination.

Regarding the content ratio of each of the azole compound according to the present invention and the second curable compound in the second resin composition, the content ratio is preferably an appropriate ratio in a range where the content of the second curable compound is 0 to 1000 times (by weight) with respect to the content of the azole compound according to the present invention, and is more preferably an appropriate ratio within a range where the content of the second curable compound is 0.01 times to 100 times (by weight) with respect to the content of the azole compound according to the present invention.

Examples of a method of polymerizing (curing) the second resin composition include a method of performing photocuring and thermal curing.

Examples of the photocuring method include a method of radiating active energy rays, and preferably a method of using a photopolymerization initiator in combination. Examples of the active energy ray include light, radiation, electromagnetic waves, and electron beams, and electron beams or light in an ultraviolet to infrared wavelength range is preferable. As the light source, for example, an ultra-high pressure mercury light source or a metal halide light source can be used in the case of irradiation with ultraviolet rays, a metal halide light source or a halogen light source can be used in the case of irradiation with visible rays, and a halogen light source can be used in the case of irradiation with infrared rays. In addition, a light source such as a laser or an LED corresponding to light emission of various wavelengths, which has been widely used in recent years, may be used.

The irradiation amount of the active energy rays can be appropriately set according to the type of the light source or the like.

As the photopolymerization initiator, a photocationic polymerization initiator can be adopted, and if necessary, a photoradical polymerization initiator can be used in combination. These may be contained in the resin composition. In photocuring, thermal curing methods may be used in combination in order to enhance production efficiency and properties of the cured product.

The photocationic polymerization initiator is not particularly limited as long as it is commonly used, and examples thereof include onium salts and organometallic complexes. As the photocationic polymerization initiator, the photocationic polymerization initiator described above can be used.

The content of the photocationic polymerization initiator in the second resin composition is preferably 0.001 wt% to 20 wt%, and more preferably 0.01 wt% to 10 wt%.

As the photoradical polymerization initiator, the photoradical polymerization initiator described above can be used.

The content of the photoradical polymerization initiator in the second resin composition is preferably 0.001 wt% to 20 wt%, and more preferably 0.01 wt% to 10 wt%.

When the second resin composition is photocured, the sensitizer described above can be used.

On the other hand, when the second resin composition is thermally cured, a thermal polymerization initiator can be used. As the thermal polymerization initiator, a thermal cationic polymerization initiator can be adopted, and the thermal cationic polymerization initiator may be contained in the resin composition.

Examples of the heating method include hot air circulation, infrared heating, and highfrequency heating. As the curing device, a closed curing furnace, a tunnel furnace capable of performing continuous curing, or the like can be used.

The heating (curing) temperature and the heating (curing) time may be appropriately set in consideration of the composition and the shape (thickness) of the resin composition as an irradiation target.

The thermal cationic polymerization initiator is not particularly limited as long as it is commonly used, and examples thereof include various onium salts such as quaternary ammonium salts, phosphonium salts, and sulfonium salts, and organometallic complexes. As the thermal cationic polymerization initiator, the above-described thermal cationic polymerization initiator can be used.

The content of the thermal cationic polymerization initiator in the second resin composition is preferably 0.001 wt% to 20 wt%, and more preferably 0.01 wt% to 10 wt%.

The second resin composition may further optionally contain the above-described additive (modifier) in a proportion of 0.01 wt% to 50 wt% with respect to the entire second resin composition (total amount) as long as the effect of the present invention is not impaired.

A method for preparing the second resin composition is not particularly limited, and the second resin composition can be prepared by measuring a predetermined amount of each of the above-described components and stirring and mixing the components. For example, after preliminary mixing, the mixture can be prepared by mixing or melt-kneading by using a roll kneader, a kneader, an extruder, or the like. If necessary, an organic solvent (viscosity-adjusting diluent) may be used.

The resin composition containing the azole compound according to the present invention (hereinafter, including the "first resin composition" and the "second resin composition") is expected to provide a cured product excellent in low thermal expansion, heat resistance, and mechanical strength. Therefore, the resin composition containing the azole compound according to the present invention is suitable as a material used for producing a coating material, an ink, an adhesive, a pressure-sensitive adhesive, a gas barrier film, a color filter, an optical film, an optical lens, a touch panel, and the like.

Examples of the application of the coating material include protection (hard coating) of a touch panel, a plastic container, a plastic sheet, a plastic film, a film-type liquid crystal element, a polarizing plate used in a liquid crystal display device, an optical component, and a building interior material (floor material, wall material, artificial marble, and the like).

Examples of the ink include a coloring ink, a printing ink, a UV ink, and an inkjet ink. These inks can be used for offset printing, flexographic printing, gravure printing, screen printing, inkjet printing, and the like.

Examples of the applications of the adhesive include semiconductor applications, optical applications, optical component applications, optical waveguide-coupling applications, optical waveguide peripheral member-fixing applications, and CD/DVD-bonding applications.

Examples of the applications of the pressure-sensitive adhesive include a pressure-sensitive adhesive tape (an adhesive tape), a pressure-sensitive adhesive sheet (an adhesive sheet), and a pressure-sensitive adhesive label (an adhesive label).

Examples of applications of the gas barrier film include electronic paper, a flexible display, an organic EL element, and an organic solar cell.

Examples of the applications of the color filter include a color liquid crystal display device (color filter on array (COA)), a color imaging element, and an organic EL display device.

Examples of the optical film include functional films such as a protective film for a polarizing plate, a film for a liquid crystal display device, such as a support film for a prism sheet or a light guide film, a hard coat film, a decorative film, and a transparent conductive film, a weather (light)-resistant film for a solar cell, a film for LED lighting or organic EL lighting, and a transparent heat-resistant film for flexible electronics.

Examples of the optical lens include lenses for microscopes, endoscopes, telescopes, cameras, glasses, and the like, lenses for laser beam printers, lenses for sensors, prism lenses, and pickup lenses for optical discs.

Examples of applications of the touch panel include personal computers, car navigation systems, mobile phones, electronic dictionaries, and OA/FA equipment.

The resin composition containing the azole compound according to the present invention is further expected to be used as a raw material or a member such as a vibration-damping material, a waterproof material, a moisture-proof material, a heat-shrinkable rubber tube, an O-ring, a gas separation membrane, a concrete protective material, a lining, a soil injection agent, a cold and heat storage material, a sealing material for a sterilization treatment device, and an oxygen permeable membrane, in addition to materials in the electric and electronic fields, such as a transparent material, an optical material, a dicing tape, an insulating material (such as an electric wire coating), a solder resist ink, a printed wiring board, a copper-clad laminate, a resin-coated copper foil, a prepreg, a high-voltage insulating material, an interlayer insulating material, a passivation film for TFTs, a gate insulating film for TFTs, an interlayer insulating film for TFTs, a transparent planarization film for TFTs, an insulating packing material, an insulating coating material, a paint, a UV powder paint, a molding material (a sheet, a film, FRP, etc.), a sealing material, a liquid crystal sealing material, a sealing material for display device, a high-heat-resistant sealing material, a potting material, an encapsulant (a semiconductor encapsulant, an electrical material encapsulant, encapsulants for organic EL or LED elements, encapsulants for various solar cells), a resist material (a liquid resist material, a colored resist material, a dry film resist material, a solder resist material, and a color filter resist material), a photo spacer material for liquid crystal cells, stereolithography, a material for solar cells, materials for fuel cells, display materials, recording materials, a photosensitive drum for copiers, and a solid electrolyte for batteries, and as an additive (modifier) blended in other resins or resin compositions (thermoplastic resin compositions, thermosetting resin compositions, and photocurable resin compositions).

### [Examples]

Hereinafter, the present invention will be specifically described with reference to Examples, but the present invention is not limited thereto.

The main raw materials used in Examples and Synthesis Examples are as follows.

### [Main Raw Materials]

- 3,5-diamino-1H-1,2,4-triazole (manufactured by Tokyo Chemical Industry Co., Ltd., refer to the chemical formula (III-1))
- 3-amino-5-methylthio-1H-1,2,4-triazole (manufactured by Tokyo Chemical Industry Co., Ltd., refer to the chemical formula (III-2))
- 3,5-dimethyl-1H-1,2,4-triazole (manufactured by Tokyo Chemical Industry Co., Ltd., refer to the chemical formula (III-3))
- 3-chloro-1H-1,2,4-triazole (manufactured by Tokyo Chemical Industry Co., Ltd., refer to the chemical formula (III-4))
- 5-amino-1H-tetrazole (manufactured by Masuda Chemical Industry Co., Ltd., refer to the chemical formula (IV-1))
- 5-ethylthio-1H-tetrazole (manufactured by Tokyo Chemical Industry Co., Ltd., refer to the chemical formula (IV-2))
- 5-phenyl-1H-tetrazole (manufactured by Tokyo Chemical Industry Co., Ltd., refer to the chemical formula (IV-3))
- 5-benzyl-1H-tetrazole (manufactured by Tokyo Chemical Industry Co., Ltd., refer to the chemical formula (IV-4))
- 4-vinylbenzyl chloride (manufactured by Tokyo Chemical Industry Co., Ltd., purity: 95.9%)
- 1H-1,2,4-triazole (manufactured by Tokyo Chemical Industry Co., Ltd.)
- 4-ethylbenzyl chloride (manufactured by Tokyo Chemical Industry Co., Ltd.)

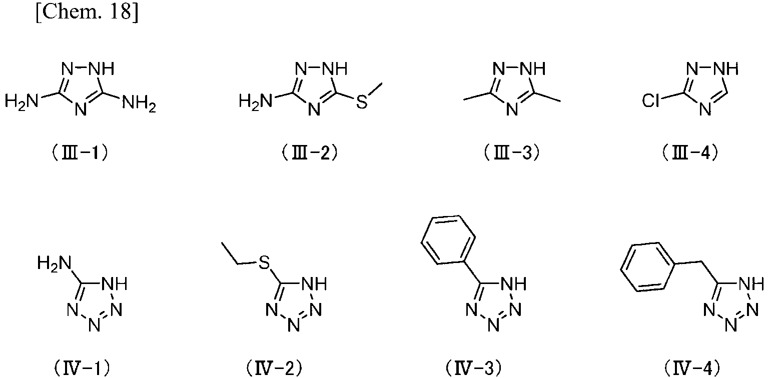

The azole compounds used in Comparative Examples (evaluation tests) are as follows.

### [Azole Compounds]

- Azole compound (1-(4-vinylbenzyl)-1H-1,2,4-triazole) represented by the chemical formula (41): synthesized according to the method in Synthesis Example 1.
- A mixture of the azole compound represented by the chemical formula (42) and the azole compound represented by the chemical formula (43) (5-amino-1-(4-ethylbenzyl)-1H-tetrazole and 5-amino-2-(4-ethylbenzyl)-2H-tetrazole): synthesized according to the method in Synthesis Example 2.

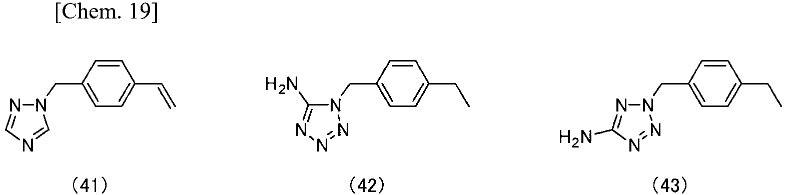

### [Synthesis Example 1]

### <Synthesis of 1-(4-vinylbenzyl)-1H-1,2,4-triazole>

A solution containing 10.0 g (145 mmol) of 1H-1,2,4-triazole and 56 mL of N,N-dimethylformamide was heated to 70°C, 28.7 g (145 mmol) of a 28% sodium methoxide methanol solution was added thereto, followed by stirring for 1 hour and then cooling to 40°C, and methanol was distilled off under reduced pressure. Subsequently, 23.0 g (145 mmol) of 4-vinylbenzyl chloride was added thereto, followed by stirring at 55°C for 17 hours. The reaction solution was cooled to room temperature, insoluble matter was filtered off, and the filtrate was distilled off under reduced pressure to obtain a brown viscous substance. Subsequently, 200 mL of water was added, followed by stirring at room temperature for 1 hour, and then the solid was collected by filtration, washed with n-hexane, and then dried to obtain 23.5 g (126.7 mmol, yield: 87.5%) of a light yellow solid.

The ¹H-NMR spectral data of the obtained light yellow solid was as follows.

¹H-NMR (DMSO-d₆) δ: 5.26 (d, 1H), 5.40 (s, 2H), 5.83 (d, 1H), 6.72 (dd, 1H), 7.25 (d, 2H), 7.46 (d, 2H), 7.98 (s, 1H), 8.66 (s, 1H).

From the ¹H-NMR spectral data, the obtained light yellow solid was identified as the azole compound in the title, which was represented by the chemical formula (41).

### [Synthesis Example 2]

### <Synthesis of Mixture of (5-amino-1-(4-ethylbenzyl)-1H-tetrazole and 5-amino-2-(4-ethylbenzyl)-2H-tetrazole)>

A solution containing 10.0 g (118 mmol) of 5-amino-1H-tetrazole and 32 mL of N,N-dimethylformamide was heated to 70°C, 40.1 g (118 mol) of a 20% sodium ethoxide ethanol solution was added thereto, followed by stirring for 1 hour and then cooling to 40°C, and ethanol was distilled off under reduced pressure. Subsequently, 18.2 g (118 mmol) of 4-ethylbenzyl chloride was added thereto, followed by stirring at 60°C for 16 hours. The reaction solution was cooled to room temperature, insoluble matter was filtered off, and the filtrate was distilled off under reduced pressure to obtain a solid. Subsequently, 250 mL of water was added thereto, followed by stirring at room temperature for 1 hour, and then the solid was collected by filtration. Further, 150 mL of n-hexane was added, followed by stirring at room temperature for 1 hour, and then a solid was collected by filtration, washed with n-hexane, and dried to obtain 20.7 g (101.8 mmol, yield: 86.6%) of a white powder.

The ¹H-NMR spectral data of the obtained white powder was as follows.

¹H-NMR (DMSO-d₆) δ: 1.16 (m, 3H), 2.57 (m, 2H), 5.32 (s, 1.2H), 5.56 (s, 0.8H), 6.01 (s, 0.8H), 6.82 (s, 1.2H), 7.19 (m, 4H).

From the ¹H-NMR spectral data, the obtained white powder was identified as a mixture of the azole compounds in the title, which were represented by the chemical formula (42) and the chemical formula (43).

### <Synthesis of Azole Compound (I)>

The azole compounds of Examples 1 to 8 were synthesized.

### [Example 1]

### <Synthesis of 3,5-di-amino-1-(4-vinylbenzyl)-1H-1,2,4-triazole>

A solution containing 54.1 g (159 mmol) of a 20% sodium ethoxide ethanol solution and 46 mL of N,N-dimethylacetamide was heated to 45°C, 15.8 g (159 mmol) of 3,5-di-amino-1H-1,2,4-triazole was added thereto, the temperature was raised to 70 °C, followed by stirring for 1 hour and then cooling to 50°C, and ethanol was distilled off under reduced pressure. Subsequently, 25.1 g (158 mmol) of 4-vinylbenzyl chloride was added thereto, followed by stirring at 50°C for 20 hours. The reaction solution was cooled to room temperature, insoluble matter was filtered off, and the filtrate was distilled off under reduced pressure to obtain a brown viscous substance. Subsequently, 200 mL of water was added thereto, followed by stirring at room temperature for 1 hour, and then the solid was collected by filtration. Further, 100 mL of ethyl acetate was added, followed by stirring at room temperature for 1 hour, and then a solid was collected by filtration, washed with ethyl acetate, and dried to obtain 17.8 g (82.7 mmol, yield: 52.3%) of a light yellow powder.

The ¹H-NMR spectral data of the obtained light yellow powder was as follows.

¹H-NMR (DMSO-d₆) δ: 4.72 (s, 2H), 4.84 (s, 2H), 5.23 (dd, 1H), 5.80 (dd, 1H), 6.00 (s, 2H), 6.70 (dd, 1H), 7.17 (d, 2H), 7.42 (d, 2H).

From the ¹H-NMR spectral data, the obtained light yellow powder was identified as the azole compound in the title, which was represented by the chemical formula (I-1-1).

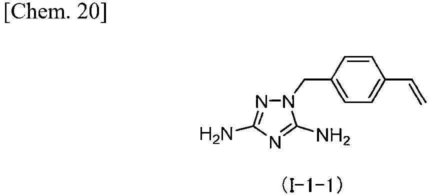

### [Example 2]

### <Synthesis of Mixture of 3-amino-5-methylthio-1-(4-vinylbenzyl)-1H-1,2,4-triazole and 5-amino-3-methylthio-1-(4-vinylbenzyl)-1H-1,2,4-triazole>

A solution containing 12.0 g (92 mmol) of 3-amino-5-methylthio-1H-1,2,4-triazole and 37 mL of N,N-dimethylformamide was heated to 70°C, 31.4 g (92 mmol) of a 20% sodium ethoxide ethanol solution was added thereto, followed by stirring for 1 hour and then cooling to 40°C, and ethanol was distilled off under reduced pressure. Subsequently, 14.6 g (92 mmol) of 4-vinylbenzyl chloride was added thereto, followed by stirring at 60°C for 21 hours. The reaction solution was cooled to room temperature, insoluble matter was filtered off, and the filtrate was distilled off under reduced pressure to obtain a brown viscous substance. Subsequently, 120 mL of water was added thereto, followed by stirring at room temperature for 1 hour, and then the solid was collected by filtration. Further, 80 mL of n-hexane was added, followed by stirring at room temperature for 1 hour, and then a solid was collected by filtration, washed with n-hexane, and dried to obtain 18.5 g (75.1 mmol, yield: 81.5%) of a brown solid.

The ¹H-NMR spectral data of the obtained brown solid was as follows.

¹H-NMR (CDCl₃) δ: 2.52 (s, 1.8H), 2.61 (s, 1.2HH), 4.52 (br, 2H), 5.03 (s, 0.8H), 5.06 (s, 1.2H), 5.26 (m, 1H), 5.75 (m, 1H), 6.69 (m, 1H), 7.19 (m, 2H), 7.38 (m, 2H).

From the ¹H-NMR spectral data, the obtained brown solid was identified as the azole compounds in the title, which were represented by the chemical formula (I-1-2) and the chemical formula (I-1-3).

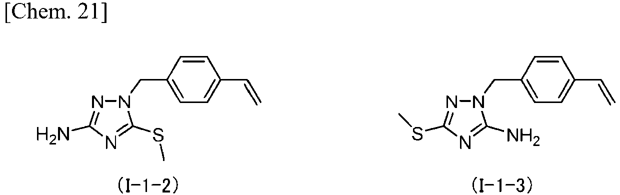

### [Example 3]

### <Synthesis of 3,5-dimethyl-1-(4-vinylbenzyl)-1H-1,2,4-triazole>

A solution containing 5.1 g (52 mmol) of 3,5-dimethyl-1H-1,2,4-triazole and 20 mL of N,N-dimethylformamide was heated to 70°C, 17.8 g (52 mmol) of a 20% sodium ethoxide ethanol solution was added thereto, followed by stirring for 1 hour and then cooling to 40°C, and ethanol was distilled off under reduced pressure. Subsequently, 8.3 g (52 mmol) of 4-vinylbenzyl chloride was added thereto, followed by stirring at 60°C for 20 hours. The reaction solution was cooled to room temperature, insoluble matter was filtered off, and the filtrate was distilled off under reduced pressure to obtain a brown liquid. Subsequently, 80 mL of water was added, followed by stirring at room temperature for 1 hour, and then, the aqueous layer was removed. Further, 40 mL of n-hexane was added, followed by stirring at room temperature for 1 hour, then the hexane layer was removed, and the resultant was concentrated under reduced pressure to obtain 6.2 g (29.1 mmol, yield: 55.4%) of a brown liquid.

The ¹H-NMR spectral data of the obtained brown liquid was as follows.

¹H-NMR (CDCl₃) δ: 2.35 (s, 6H), 5.19 (s, 2H), 5.27 (d, 1H), 5.74 (d, 1H), 6.69 (dd, 1H), 7.12 (d, 2H), 7.38 (d, 2H).

From the ¹H-NMR spectral data, the obtained brown liquid was identified as the azole compound in the title, which was represented by the chemical formula (I-1-4).

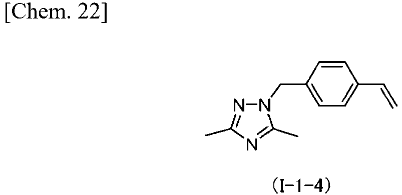

### [Example 4]

### <Synthesis of Mixture of 3-chloro-1-(4-vinylbenzyl)-1H-1,2,4-triazole and 5-chloro-1-(4-vinylbenzyl)-1H-1,2,4-triazole>

A solution containing 5.0 g (48 mmol) of 3-chloro-1H-1,2,4-triazole and 17 mL of N,N-dimethylformamide was heated to 70°C, 16.4 g (48 mmol) of a 20% sodium ethoxide ethanol solution was added thereto, followed by stirring for 1 hour and then cooling to 40°C, and ethanol was distilled off under reduced pressure. Subsequently, 7.6 g (48 mmol) of 4-vinylbenzyl chloride was added thereto, followed by stirring at 60°C for 20 hours. The reaction solution was cooled to room temperature, insoluble matter was filtered off, and the filtrate was distilled off under reduced pressure to obtain a brown viscous substance. Subsequently, 80 mL of water was added, followed by stirring at room temperature for 1 hour, and then, the aqueous layer was removed. Further, 40 mL of n-hexane was added, followed by stirring at room temperature for 1 hour, then the hexane layer was removed, and the resultant was concentrated under reduced pressure to obtain 9.2 g (41.9 mmol, yield: 86.9%) of a brown viscous substance.

The ¹H-NMR spectral data of the obtained brown viscous substance was as follows.

¹H-NMR (CDCl₃) δ: 5.25 (s, 1.6H), 5.29 (s, 0.4H), 5.32 (m, 1H), 5.80 (m, 1H), 6.71 (m, 1H), 7.25 (m, 2H), 7.43 (m, 2H), 7.86 (s, 0.2H), 7.91 (s, 0.8H).

From the ¹H-NMR spectral data, the obtained brown viscous substance was identified as the azole compounds in the title, which were represented by the chemical formula (I-1-5) and the chemical formula (I-1-6).

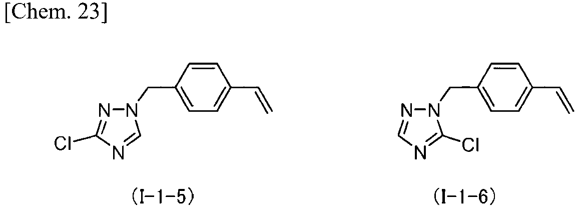

### [Example 5]

### <Synthesis of Mixture of 5-amino-1-(4-vinylbenzyl)-1H-tetrazole and 5-amino-2-(4-vinylbenzyl)-2H-tetrazole>

A solution containing 13.4 g (158 mmol) of 5-amino-1H-tetrazole and 50 mL of N,N-dimethylacetamide was heated to 70°C, 54.1 g (159 mol) of a 20% sodium ethoxide ethanol solution was added thereto, followed by stirring for 1 hour and then cooling to 50°C, and ethanol was distilled off under reduced pressure. Subsequently, 25.1 g (158 mmol) of 4-vinylbenzyl chloride was added thereto, followed by stirring at 50°C for 20 hours. The reaction solution was cooled to room temperature, insoluble matter was filtered off, and the filtrate was distilled off under reduced pressure to obtain a solid. Subsequently, 120 mL of water was added, followed by stirring at room temperature for 1 hour, and then the solid was collected by filtration, washed with water, and then dried to obtain 28.6 g (142.1 mmol, yield: 90.0%) of a light peach powder.

The ¹H-NMR spectral data of the obtained light peach powder was as follows.

¹H-NMR (DMSO-d₆) δ: 5.25 (dd, 0.45H), 5.28 (dd, 0.55H), 5.36 (s, 0.90H), 5.60 (s, 1.10H), 5.81 (dd, 0.45H), 5.85 (dd, 0.55H), 6.04 (s, 0.90H), 6.70 (dd, 0.55H), 6.72 (dd, 0.45H), 6.86 (s, 1.10H), 7.22 (d, 1.10H), 7.29 (d, 0.90H), 7.46 (d, 1.10H), 7.48(d, 0.90H).

From the ¹H-NMR spectral data, the obtained light peach powder was identified as a mixture of the azole compounds in the title, which were represented by the chemical formula (I-2-1) and the chemical formula (I-3-1).

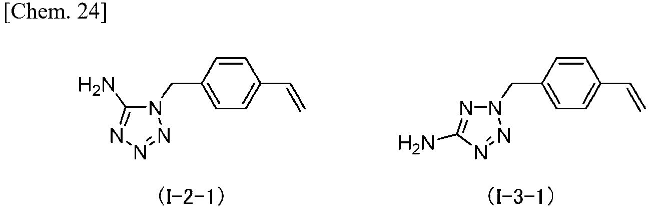

### [Example 6]

### <Synthesis of Mixture of 5-ethylthio-1-(4-vinylbenzyl)-1H-tetrazole and 5-ethylthio-2-(4-vinylbenzyl)-2H-tetrazole>

A solution containing 10.0 g (77 mmol) of 5-ethylthio-1H-tetrazole and 37 mL of N,N-dimethylformamide was heated to 70°C, 26.3 g (77 mol) of a 20% sodium ethoxide ethanol solution was added thereto, followed by stirring for 1 hour and then cooling to 40°C, and ethanol was distilled off under reduced pressure. Subsequently, 12.2 g (77 mmol) of 4-vinylbenzyl chloride was added thereto, followed by stirring at 55°C for 20 hours. The reaction solution was cooled to room temperature, insoluble matter was filtered off, and the filtrate was distilled off under reduced pressure to obtain a brown liquid. Subsequently, 100 mL of water was added thereto, followed by stirring at room temperature for 1 hour, and then the aqueous layer was removed. Further, 60 mL of n-hexane was added, followed by stirring at room temperature for 1 hour, then the hexane layer was removed, and the resultant was concentrated under reduced pressure to obtain 17.1 g (69.4 mmol, yield: 90.2%) of a brown liquid.

The ¹H-NMR spectral data of the obtained brown liquid was as follows.

¹H-NMR (CDCl₃) δ: 1.41 (m, 3H), 3.18 (q, 1.2H), 3.32 (q, 0.8H), 5.28 (d, 1H), 5.39 (s, 0.8H), 5.68 (s, 1.2H), 5.78 (d, 1H), 6.69 (m, 1H), 7.33 (m, 4H).

From the ¹H-NMR spectral data, the obtained brown liquid was identified as the azole compounds in the title, which were represented by the chemical formula (I-2-2) and the chemical formula (I-3-2).

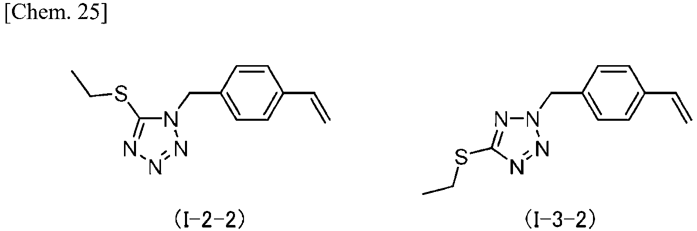

### [Example 7]

### <Synthesis of Mixture of 5-phenyl-1-(4-vinylbenzyl)-1H-tetrazole and 5-phenyl-2-(4-vinylbenzyl)-2H-tetrazole>

A solution containing 12.0 g (82 mmol) of 5-phenyl-1H-tetrazole and 37 mL of N,N-dimethylformamide was heated to 70°C, 28.0 g (82 mol) of a 20% sodium ethoxide ethanol solution was added thereto, followed by stirring for 1 hour and then cooling to 40°C, and ethanol was distilled off under reduced pressure. Subsequently, 13.0 g (82 mmol) of 4-vinylbenzyl chloride was added thereto, followed by stirring at 55°C for 20 hours. The reaction solution was cooled to room temperature, insoluble matter was filtered off, and the filtrate was distilled off under reduced pressure to obtain a reddish brown viscous substance. Subsequently, 120 mL of water was added thereto, followed by stirring at room temperature for 1 hour, and then the aqueous layer was removed. Further, 40 mL of n-hexane was added, followed by stirring at room temperature for 1 hour, then the hexane layer was removed, and the resultant was concentrated under reduced pressure to obtain 20.3 g (77.4 mmol, yield: 94.4%) of a reddish brown viscous substance.

The ¹H-NMR spectral data of the obtained reddish brown viscous substance was as follows.

¹H-NMR (CDCl₃) δ: 5.27 (m, 1H), 5.75 (d, 1H), 5.78 (s, 2H), 6.69 (m, 1H), 7.48 (m, 7H), 8.13 (m, 2H).

From the ¹H-NMR spectral data, the obtained reddish brown viscous substance was identified as the azole compounds in the title, which were represented by the chemical formula (I-2-3) and the chemical formula (I-3-3).

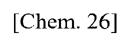

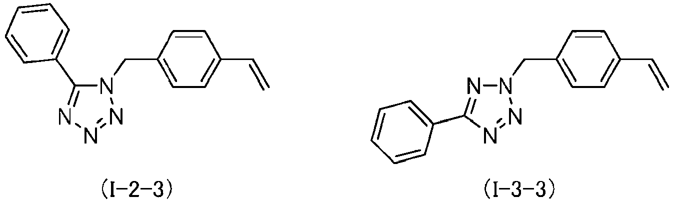

### [Example 8]

### <Synthesis of Mixture of 5-benzyl-1-(4-vinylbenzyl)-1H-tetrazole and 5-benzyl-2-(4-vinylbenzyl)-2H-tetrazole>

A solution containing 12.0 g (75 mmol) of 5-benzyl-1H-tetrazole and 37 mL of N,N-dimethylformamide was heated to 70°C, 25.5 g (75 mol) of a 20% sodium ethoxide ethanol solution was added thereto, followed by stirring for 1 hour and then cooling to 40°C, and ethanol was distilled off under reduced pressure. Subsequently, 11.9 g (75 mmol) of 4-vinylbenzyl chloride was added thereto, followed by stirring at 55°C for 20 hours. The reaction solution was cooled to room temperature, insoluble matter was filtered off, and the filtrate was distilled off under reduced pressure to obtain a brown liquid. Subsequently, 120 mL of water was added thereto, followed by stirring at room temperature for 1 hour, and then the aqueous layer was removed. Further, 60 mL of n-hexane was added, followed by stirring at room temperature for 1 hour, then the hexane layer was removed, and the resultant was concentrated under reduced pressure to obtain 20.1 g (72.7 mmol, yield: 97.0%) of a brown liquid.

The ¹H-NMR spectral data of the obtained brown liquid was as follows.

¹H-NMR (CDCl₃) δ: 4.15 (s, 1H), 4.21 (s, 1H), 5.28 (m, 1H), 5.29 (s, 1H), 5.68 (s, 1H), 5.75 (d, 1H), 6.68 (m, 1H), 6.95 (m, 1H), 7.05 (m, 1H), 7.30 (m, 7H).

From the ¹H-NMR spectral data, the obtained brown liquid was identified as the azole compounds in the title, which were represented by the chemical formula (I-2-4) and the chemical formula (I-3-4).

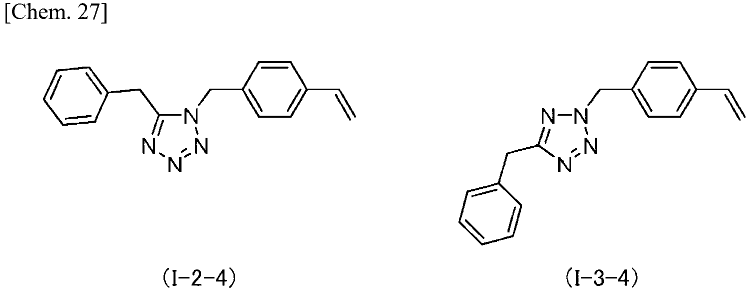

### <Adhesion Test>

The surface treatment liquids prepared in Examples 9 to 11 and Comparative Examples 2 and 3 below were evaluated for adhesion as follows.

### [Evaluation Test of adhesion]

### (1) Metal

An electrolytic copper foil (thickness: 35 µm) was used as the metal.

### (2) Surface Treatment of Metal

The copper foil was treated according to the following steps a and b.
a. Acid cleaning (with potassium persulfate soft etching agent for 30 seconds (30°C)) and washing with water were performed.
b. The surface treatment liquid was applied with a brush, and drying was performed for 1 minute (100°C).

### (3) Adhering between Metal and Resin

A dry film type solder resist (SR1-A (manufactured by Taiyo Holdings Co., Ltd., thickness: 15 µm)) was laminated and plane-pressed on an S surface of the treated copper foil, and then subjected to UV exposure and post-curing (160°C for 60 minutes) to produce a copper-clad laminate including an insulating resin layer.

### (4) Evaluation of Adhesion

For this copper-clad laminate, a test piece having a width of 10 mm was prepared in accordance with "JIS C6481 (1996)" after reflow heating (peak temperature: 260°C, air) was performed two times, and the peel strength (kN/m) of the copper foil was measured.

### [Example 9]

To 1 g of the azole compound obtained in Example 2 as a coupling agent component was added 99 g of methanol, followed by stirring at room temperature for 1 hour, to prepare a surface treatment liquid.

### [Examples 10 and 11]

Surface treatment liquids were prepared in the same manner as in Example 9 except that the azole compound obtained in Example 5 or the azole compound obtained in Example 6 was used instead of the azole compound obtained in Example 2.

### [Comparative Example 1]

The copper foil subjected to only step (2) a. of the evaluation test of adhesion was subjected to the evaluation test of adhesion.

### [Comparative Examples 2 and 3]

Surface treatment liquids were prepared in the same manner as in Example 9 except that the azole compound represented by the chemical formula (41) or a mixture of the azole compound represented by the chemical formula (42) and the azole compound represented by the chemical formula (43) was used instead of the azole compound obtained in Example 2.

Each of the surface treatment liquids prepared above was subjected to the evaluation test of adhesion. The obtained test results are shown in Table 1.

### [Table 1]

**Table 1**

| | | Coupling agent | Peel strength (kN/m) |
|---|---|---|---|
| Examples | 9 | Azole compound in Example 2 | 0.61 |
| | 10 | Azole compound in Example 5 | 0.24 |
| | 11 | Azole compound in Example 6 | 0.14 |
| Comparative Examples | 1 | No | 0.12 |
| | 2 | Azole compound (41) | 0.06 |
| | 3 | Mixture of azole compound (42) and azole compound (43) | 0.03 |

From the results of Table 1, it was found that the surface treatment liquid according to the present invention (the surface treatment liquids in Examples 9 to 11) had a higher peel strength than Comparative Examples 1 to 3, and was excellent in adhesion (adhesiveness) between a metal and a resin.

Although the present invention has been described in detail with reference to specific embodiments, it is apparent to those skilled in the art that various changes and modifications can be made without departing from the spirit and scope of the present invention. The present application is based on Japanese Patent Application (No. 2023-088281) filed on May 30, 2023, the contents of which are incorporated herein by reference.

### INDUSTRIAL APPLICABILITY

The azole compound according to the present invention can be used as a coupling agent having a function of preventing a metal from rusting, which is a feature of an azole compound, and a function of curing a resin such as an epoxy resin or a urethane resin. Therefore, the azole compound is expected to be used for a composite material such as a printed wiring board produced by combining a large number of different types of materials.

In addition, according to the present invention, it can be expected that the adhesion (adhesiveness) of a metal, an inorganic material, and a resin material is enhanced, and therefore, the surface of the base material can be maintained in a smooth state without being roughened. Therefore, since the present invention can greatly contribute to the implementation of miniaturization, thinning, high frequency, high density, and the like of a multilayer printed wiring board, industrial applicability is great.

## Claims

1. An azole compound represented by the chemical formula (I): 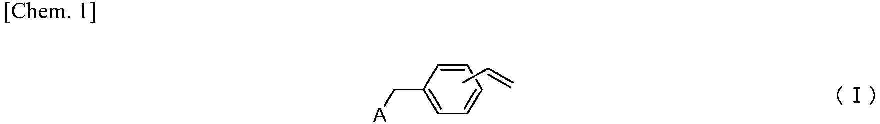
(in the formula (I), A represents a group represented by any of the formulas (1) to (3)): 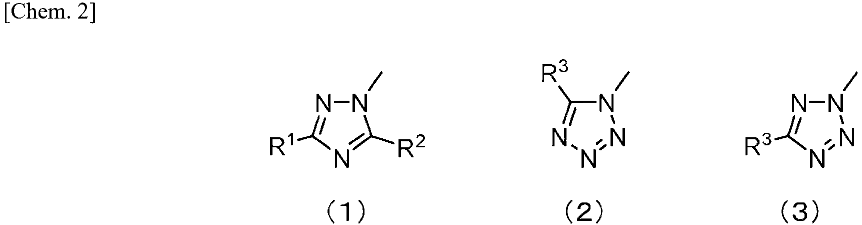
(in the formula (1), R¹ and R² are the same as or different from each other, and represent a hydrogen atom, a linear or branched alkyl group having 1 to 12 carbon atoms, an aryl group, an aralkyl group, an amino group which may have a substituent, a hydroxy group, a cyano group, a halogen atom, an alkylthio group having 1 to 6 carbon atoms, a group represented by the formula (11), or a group represented by the formula (12), in the formula (2) and the formula (3), R³ represents a hydrogen atom, a linear or branched alkyl group having 1 to 12 carbon atoms, an aryl group, an aralkyl group, an amino group which may have a substituent, a hydroxy group, a cyano group, a halogen atom, an alkylthio group having 1 to 6 carbon atoms, a group represented by the formula (21), or a group represented by the formula (31), here, the case where R¹ and R² are hydrogen atoms at the same time, the case where R¹ and R² are groups represented by the formula (11) at the same time, the case where R¹ and R² are groups represented by the formula (12) at the same time, and the case where R¹ and R² are groups represented by the formula (11) and the formula (12) are excluded), 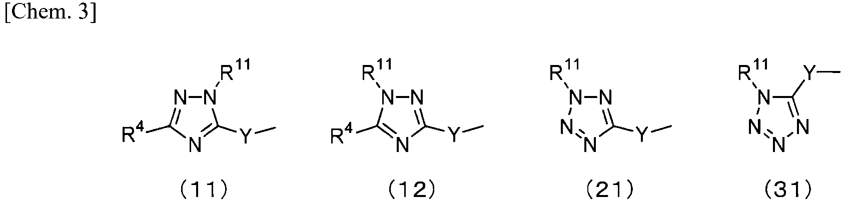
(in the formula (11) and the formula (12), R⁴ represents a hydrogen atom, a linear or branched alkyl group having 1 to 12 carbon atoms, an aryl group, an aralkyl group, an amino group which may have a substituent, a hydroxy group, a cyano group, a halogen atom, or an alkylthio group having 1 to 6 carbon atoms, in the formula (11), the formula (12), the formula (21), and the formula (31), R¹¹ represents a hydrogen atom or a group represented by the formula (4), Y represents a phenylene group, -NH-, or a group represented by -(CH₂)ₙ-, where n represents an integer of 0 to 12).

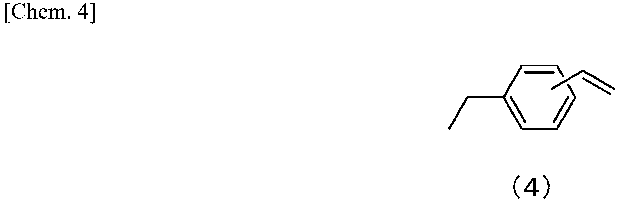

2. A method for synthesizing the azole compound according to claim 1 comprising: reacting a styryl compound represented by the chemical formula (II) and a triazole compound represented by the chemical formula (III) or a tetrazole compound represented by the chemical formula (IV), 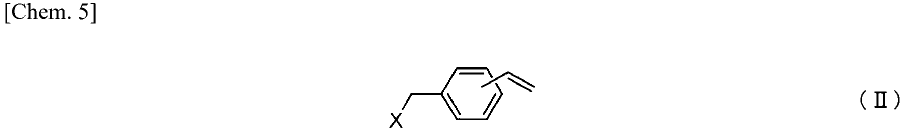
(in the formula (II), X represents a fluorine atom, a chlorine atom, a bromine atom, or an iodine atom), 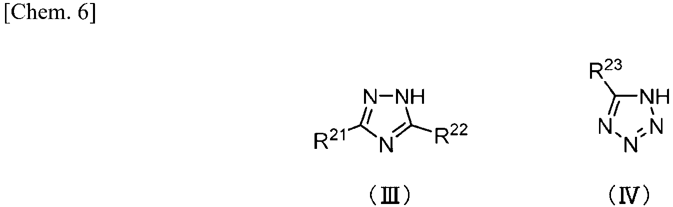
(in the formula (III), R²¹ and R²² are the same as or different from each other, and represent a hydrogen atom, a linear or branched alkyl group having 1 to 12 carbon atoms, an aryl group, an aralkyl group, an amino group which may have a substituent, a hydroxy group, a cyano group, a halogen atom, an alkylthio group having 1 to 6 carbon atoms, a group represented by the formula (13), or a group represented by the formula (14), in the formula (IV), R²³ represents a hydrogen atom, a linear or branched alkyl group having 1 to 12 carbon atoms, an aryl group, an aralkyl group, an amino group which may have a substituent, a hydroxy group, a cyano group, a halogen atom, an alkylthio group having 1 to 6 carbon atoms, a group represented by the formula (22), or a group represented by the formula (32), here, the case where R²¹ and R²² are hydrogen atoms at the same time, the case where R²¹ and R²² are groups represented by the formula (13) at the same time, the case where R²¹ and R²² are groups represented by the formula (14) at the same time, and the case where R²¹ and R²² are groups represented by the formula (13) and the formula (14) are excluded), 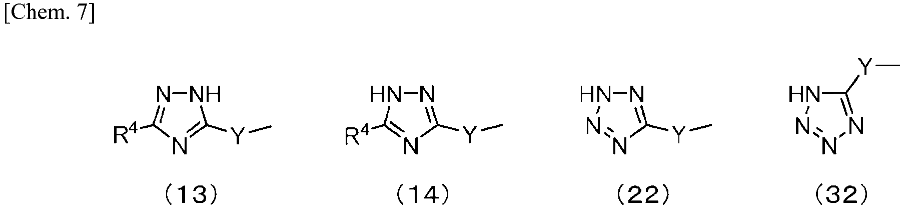
(R⁴ in the formula (13) and the formula (14), and Y in the formula (13), the formula (14), the formula (22), and the formula (32) are the same as those described above).

3. A coupling agent comprising the azole compound according to claim 1 as a component.

4. A surface treatment liquid comprising the azole compound according to claim 1.

5. The surface treatment liquid according to claim 4, which is used for treating a surface of at least one selected from the group consisting of a metal, an inorganic material, and a resin material.

6. The surface treatment liquid according to claim 4, which is used for adhering two materials selected from the group consisting of a metal, an inorganic material, and a resin material to each other.

7. The surface treatment liquid according to claim 5 or 6, wherein the metal is at least one selected from the group consisting of copper, aluminum, titanium, nickel, tin, iron, silver, gold, and alloys thereof.

8. A surface treatment method comprising: bringing the surface treatment liquid according to claim 4 into contact with a surface of at least one selected from the group consisting of a metal, an inorganic material, and a resin material.

9. The surface treatment method according to claim 8, wherein the metal is at least one selected from the group consisting of copper, aluminum, titanium, nickel, tin, iron, silver, gold, and alloys thereof.

10. The surface treatment method according to claim 9, wherein the metal is copper or a copper alloy.

11. The surface treatment method according to claim 10, wherein an aqueous solution containing a copper ion is brought into contact with a surface of the copper or the copper alloy before the surface treatment liquid is brought into contact with the surface of the copper or the copper alloy.

12. The surface treatment method according to claim 10 or 11, wherein after the surface treatment liquid is brought into contact with the surface of the copper or the copper alloy, an acidic aqueous solution or an alkaline aqueous solution is brought into contact with the surface of the copper or the copper alloy.

13. An adhesion method comprising: bringing the surface treatment liquid according to claim 4 into contact with at least one selected from the group consisting of a metal, an inorganic material, and a resin material to form a chemical conversion film on the at least one; and adhering the at least one and another one of the group to each other via the chemical conversion film.

14. A method for adhering a metal and a resin material to each other, comprising: bringing the surface treatment liquid according to claim 4 into contact with at least one of the metal and the resin material to form a chemical conversion film on the at least one; and adhering the metal and the resin material to each other via the chemical conversion film.

15. A printed wiring board comprising two materials selected from the group consisting of a metal, an inorganic material, and a resin material, wherein the two materials adhere to each other via a chemical conversion film formed of the surface treatment liquid according to claim 4.

16. A semiconductor wafer comprising two materials selected from the group consisting of a metal, an inorganic material, and a resin material, wherein the two materials adhere to each other via a chemical conversion film formed of the surface treatment liquid according to claim 4.

17. An insulating composition comprising the coupling agent according to claim 3 and a resin material or an inorganic material.

18. An insulating material comprising the insulating composition according to claim 17.

19. A printed wiring board comprising an insulating layer obtained from the insulating composition according to claim 17.

20. A semiconductor wafer comprising an insulating layer obtained from the insulating composition according to claim 17.
